(19) Europäisches Patentamt
European Patent Office
Office européen des brevets

(11) Publication number: **0 188 887 B1**

(12) # EUROPEAN PATENT SPECIFICATION

(45) Date of publication of patent specification: **02.05.91**

(51) Int. Cl.5: **C07D 295/02**, C07D 265/30, C07D 211/14, C07D 211/22, C07C 211/27, C07F 7/10, C07C 61/39, C07C 69/753, C07C 33/34

(21) Application number: **85309054.6**

(22) Date of filing: **12.12.85**

The file contains technical information submitted after the application was filed and not included in this specification

(54) **Tertiary amine compounds.**

(30) Priority: **17.01.85 GB 8501169**
**16.05.85 GB 8512400**
**03.07.85 GB 8516804**
**16.08.85 GB 8520592**
**29.11.85 GB 8529482**

(43) Date of publication of application:
**30.07.86 Bulletin 86/31**

(45) Publication of the grant of the patent:
**02.05.91 Bulletin 91/18**

(84) Designated Contracting States:
**AT BE CH DE FR GB IT LI NL SE**

(56) References cited:
**EP-A- 0 095 696**
**AT-B- 273 066**
**GB-A- 1 589 253**
**EP-A- 0 136 451**
**FR-A- 1 349 124**
**GB-A- 1 591 267**

**PATENT ABSTRACTS OF JAPAN, unexamined applications, field C, vol. 5, no. 49, 8 Apr 1981 THE PATENT OFFICE JAPANESE GOVERNMENT**

(73) Proprietor: **IMPERIAL CHEMICAL INDUSTRIES PLC**
**Imperial Chemical House, Millbank**
**London SW1P 3JF(GB)**

(72) Inventor: **Worthington, Paul Anthony**
**4 Oakhurst Road Maidenhead Court Park**
**Maidenhead Berkshire(GB)**
Inventor: **Sugavanam, Balasubramanyan**
**1020 Ausstellungs Strasse**
**A-18 Vienne(AT)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD(GB)**

**EP 0 188 887 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol 82, no. 13, 31 March 1975, Columbus, Ohio(USA) T SHONO et al. "Small ring compounds. XXIX.Thermal decomposition of tert. butyl(trans-2-substituted cycloporopyl) per-acetates", p. 479, abstract 85835s

## Description

This invention relates to cyclopropane containing tertiary amines useful as fungicides, to a process for preparing them, to fungicidal compositions containing them, and to methods of using them to combat fungi, especially fungal infections in plants.

There are described in AT-B-273066 certain phenylcyclopropane derivatives and their salts and these, depending on the substituents chosen, possess a wide range of pharmacological properties including antimycotic effects. There is no guidance, however, as to which substituents will produce such anti-mycotic effects.

The invention provides a method of combating plant fungi which comprises applying to a plant, to seed of a plant, or to the locus of a plant or seed a compound having the general formula (I) :

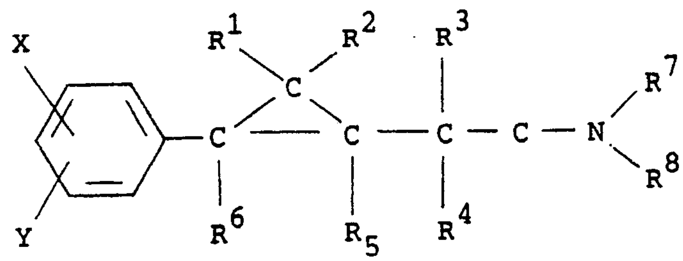

or a stereoisomer or acid addition salt thereof, wherein $R^1$, $R^2$, $R^3$, $R^4$, $R^5$ and $R^6$, which may be the same or different, represent a hydrogen atom, an alkyl group containing from 1 to 4 carbon atoms, or a halogen atom; $R^7$ and $R^8$, which may be the same or different, represent a hydrogen atom or an alkyl group containing from 1 to 4 carbon atoms or together form a ring which may contain oxygen, nitrogen or sulphur as an additional heteroatom, and which ring may be optionally substituted with one or more $C_{1-4}$ alkyl groups, or a phenyl or an hydroxyalkyl group; X and Y which may be the same or different, represent a hydrogen or a halogen atom, or an alkyl group containing from 1 to 6 carbons atoms, a cycloalkyl group containing from 3 to 6 carbon atoms, an alkenyl or alkynyl group each containing 2 to 6 carbon atoms, a phenyl group, a benzyl group, an alkoxy group containing form 1 to 6 carbon atoms, a phenoxy group, a benzyloxy group, or a

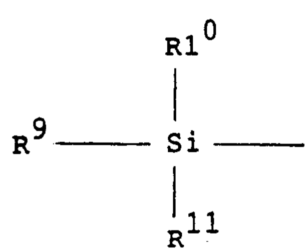

group wherein $R^9$, $R^{10}$ and $R^{11}$, which may be the same or different, represent an alkyl group containing up to 4 carbon atoms, an aalkenyl or alkynyl group each containing from 2 to 4 carbon atoms, a cycloalkyl group containing from 3 to 6 carbon atoms, or a phenyl group.

The compounds used in the invention are generally obtained in the form of mixtures of geometric isomers. However, these and other mixtures of optical isomers can be separated into individual isomers by methods in the art and such isomers consititute a part of the present invention.

When $R^7$ and $R^8$, together with the adjacent N-atom, represent a heterocyclic ring this may be, for example, a piperidine, morpholine, thiomorpholine, pyrrolidine or piperazine ring and any of these rings may bear substituents such as one or more $C_{1-4}$ alkyl groups or a phenyl group, or an hydroxyalkyl group.

The alkyl and alkoxy groups, especially for X and Y, but also for $R^1$ to $R^8$ and $R^9$ to $R^{11}$ when alkyl, may be straight or branched chain groups having 1 to 6 (for X and Y), and 1 to 4, (for $R^1$ to $R^{11}$) carbon atoms; examples are methyl, ethyl, propyl (n - or iso -propyl) and butyl (n -, sec -, iso - or t -butyl) and the corresponding alkoxy groups. Cycloalkyl groups for X and Y may be cyclopropyl, cylobutyl, cyclopentyl or cyclohexyl.

The halogen atoms which $R^1$ to $R^6$ and X and Y may represent may be fluorine, chlorine, or bromine.

The alkenyl and alkynyl groups for X and Y may contain from 2 to 6 carbon atoms and for $R^9$, $R^{10}$ and

$R^{11}$ from 2 to 4 carbon atoms.

Example of X and Y when these are aryl, aralkyl, aryloxy or aralkoxy groups are phenyl, benzyl, phenoxy and benzyloxy. These rings may be substituted with halogen (e.g. fluorine, cholorine or bromine), $C_{1-6}$ alkyl [e.g. methyl, ethyl, propyl (n - or iso -propyl) and butyl (n -, sec -, iso or t -butyl)], $C_{1-6}$ alkoxy (e.g. methoxy, ethoxy, propoxy and butoxy) halo-$C_{1-6}$-alkoxy (e.g. trifluoro-methoxy), halo-$C_{1-6}$-alkyl (e.g. trifluoromethyl), nitro, phenyl and phenoxy. The phenyl ring may thus be unsubstituted or substituted with ring substituents as defined above. The X and Y substitutents may be at the 2-, 3- or 4-positions of the phenyl ring and the 4-position is preferred. The acid additions salts can be salts with inorganic or organic acids e.g. hydrochloric, nitric, sulphuric, acetic, 4-toluene-sulphonic or oxalic acid.

In a further aspect the invention provides compounds general formula (I) :

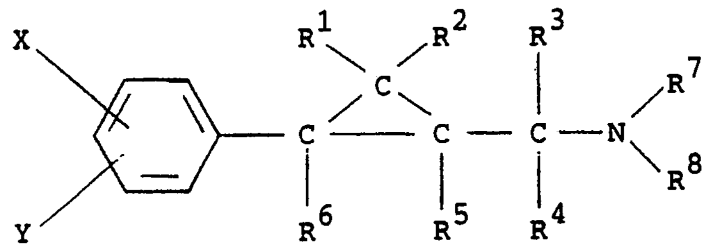

(I)

and stereoisomers and acid addition salts thereof, wherein $R^1$ and $R^2$ are hydrogen, halogen or $C_{1-4}$ alkyl; $R^7$ and $R^8$ together with the adjacent N-atom, represent a piperidine, morpholine, thiomorpholine or piperazine ring which may be substituted with $C_{1-4}$ alkyl, phenyl or hydroxymethyl; and X and Y are $C_{1-4}$ alkyl, phenyl, phenoxy, benzyl, benzyloxy or a

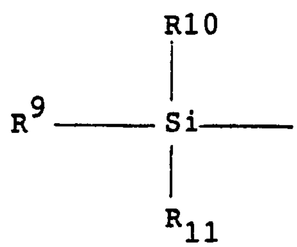

group wherein $R^9$, $R^{10}$ and $R^{11}$ are $C_{1-4}$ alkyl.

More especially the invention provides compounds of general formula :

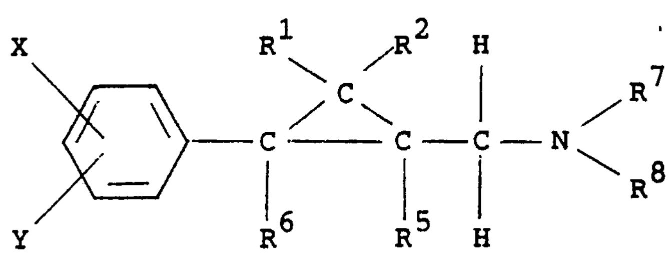

and steroisomers and acid addition salts thereof, wherein $R^1$ and $R^2$ are hydrogen, methyl or chlorine; $R^5$ and $R^6$ are hydrogen or methyl; $R^6$ and $R^7$ are piperidine, 3,5-dimethyl piperidine, morpholine, 2,6-dimethylmorpholine, 4-phenyl piperidine, 3-hydroxymethyl piperidine, thiomorpholine, piperazine, or 4-phenyl-piperazine; and X and Y are substituents at the 3- or 4- positions of the phenyl ring and one of which is hydrogen or $C_{1-4}$ alkyl whilst the other is hydrogen, $C_{1-4}$ alkyl, phenyl, phenoxy, benzyl, benzyloxy, or $C_{1-4}$ trialkylsilyl, provided that X and Y are not both hydrogen.

The invention further provides the specific compounds set out below in Talbe I; and Examples 1 to 6; and stereoisomers and acid addition salts thereof.

The invention particularly provides the compounds having the structures below :

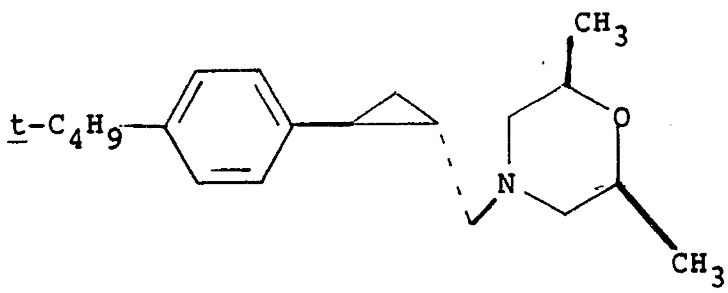

(Compound No. 4 of Table I)

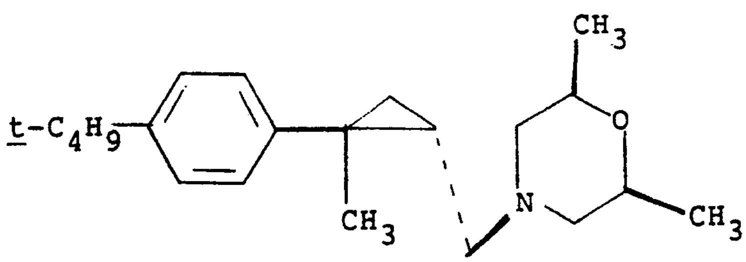

(Compound No. 15 of Table I)

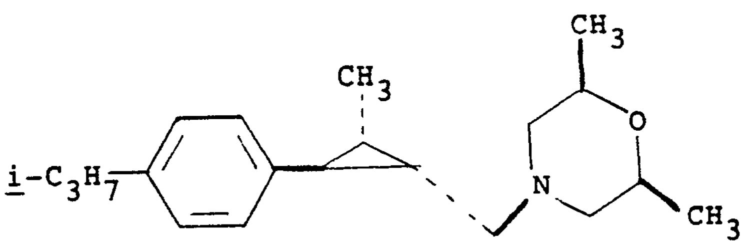

(Compound No. 94 of Table I)

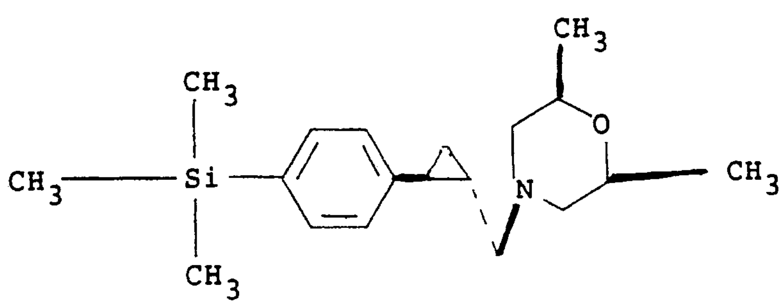

(Compiound No. 133 of Table I)

Examples of the compounds of the invention are shown in Table I. These conform to formula (I). The $^{1}H$ nmr chemical shifts of parts of these structures have been recorded in order to identify the various compounds.

TABLE I

| Compound No. | X | Y | $R^1$ | $R^2$ | $R^3$ | $R^4$ | $R^5$ | $R^6$ | 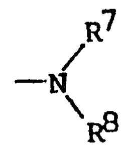 | m.p/b.p (°C) | Comments | $^1H$ nmr shifts (ppm from TMS) |
|---|---|---|---|---|---|---|---|---|---|---|---|---|
| 1 | 4-t-$C_4H_9$ | H | H | H | H | H | H | H | 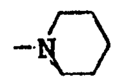 | oil | cis:trans | 2.3-2.6, 4Hm* |
| 2 | 4-t-$C_4H_9$ | H | H | H | H | H | H | H | 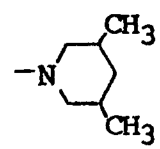 | oil | cis:trans Ar/$CH_2$=1:9 | 1.1-1.3, 9Hs (t-$C_4H_9$) |
| 3 | 4-t-$C_4H_9$ | H | H | H | H | H | H | H | 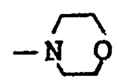 | oil | trans Ar/$CH_2$ | 3.6-3.8, 4Hm+ |
| 4 | 4-t-$C_4H_9$ | H | H | H | H | H | H | H | 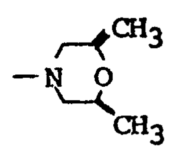 | oil | trans Ar/$CH_2$ | 3.5-3.9, 2Hm+ |
| 5 | 4-t-$C_4H_9$ | H | H | H | H | H | H | H | 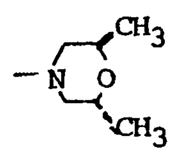 | oil | trans Ar/$CH_2$ | 3.9-4.2, 2Hm+ |

TABLE I (cont)

| Compound No. | X | Y | $R^1$ | $R^2$ | $R^3$ | $R^4$ | $R^5$ | $R^6$ | 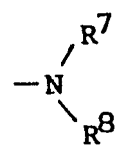 | m.p/b.p (°C) | Comments | $^1H$ nmr shifts (ppm from TMS) |
|---|---|---|---|---|---|---|---|---|---|---|---|---|
| 6 | 4-t-$C_4H_9$ | H | H | H | H | H | H | H | 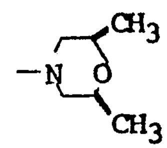 | oil | cis Ar/$CH_2$ | 3.4-3.8, 2Hm[+] |
| 7 | 4-t-$C_4H_9$ | H | H | H | H | H | H | H | 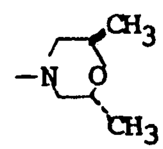 | oil | cis Ar/$CH_2$ | 3.8-4.1, 2Hm[+] |
| 8 | 4-t-$C_4H_9$ | H | $CH_3$ | $CH_3$ | H | H | H | H | 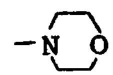 | oil | trans Ar/$CH_2$ | |
| 9 | 4-t-$C_4H_9$ | H | $CH_3$ | $CH_3$ | H | H | H | H | 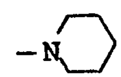 | oil | trans Ar/$CH_2$ | |
| 10 | 4-t-$C_4H_9$ | H | $CH_3$ | $CH_3$ | H | H | H | H | 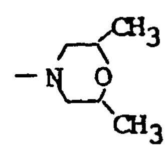 | oil | trans Ar/$CH_2$ | |

TABLE I (cont)

| Compound No. | X | Y | $R^1$ | $R^2$ | $R^3$ | $R^4$ | $R^5$ | $R^6$ | 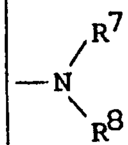 | m.p/b.p (°C) | Comments | $^1H$ nmr shifts (ppm from TMS) |
|---|---|---|---|---|---|---|---|---|---|---|---|---|
| 11 | 4-t-$C_4H_9$ | H | $CH_3$ | H | H | H | H | H | 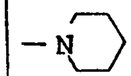 | oil | trans Ar/$CH_3$<br>trans Ar/$CH_2$ | 2.0-2.6, 4Hm* |
| 12 | 4-t-$C_4H_9$ | H | $CH_3$ | H | H | H | H | H | 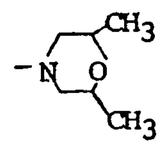 | oil | trans Ar/$CH_3$<br>trans Ar/$CH_2$ | 3.6-3.8, 2Hm$^+$ |
| 13 | 4-t-$C_4H_9$ | H | H | H | H | H | H | $CH_3$ | 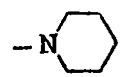 | oil | trans Ar/$CH_2$ | 2.0-2.6, 4Hm* |
| 14 | 4-t-$C_4H_9$ | H | H | H | H | H | H | $CH_3$ | 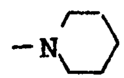 | oil | cis Ar/$CH_2$ | |
| 15 | 4-t-$C_4H_9$ | H | H | H | H | H | H | $CH_3$ | 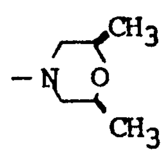 | oil | trans Ar/$CH_2$ | 3.65-3.75, 2Hm$^+$ |

TABLE I (cont)

| Compound No. | X | Y | $R^1$ | $R^2$ | $R^3$ | $R^4$ | $R^5$ | $R^6$ | 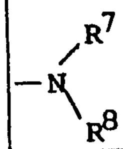 | m.p/b.p (°C) | Comments | $^1H$ nmr shifts (ppm from TMS) |
|---|---|---|---|---|---|---|---|---|---|---|---|---|
| 16 | 4-t-$C_4H_9$ | H | H | H | H | H | H | $CH_3$ | 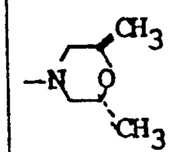 | oil | trans Ar/$CH_2$ | 3.80-4.22,2Hm+ |
| 17 | 4-t-$C_4H_9$ | H | H | H | H | H | H | $CH_3$ | 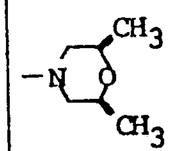 | oil | cis Ar/$CH_2$ | 3.62-3.71,2Hm+ |
| 18 | 4-t-$C_4H_9$ | H | H | H | H | H | H | $CH_3$ | 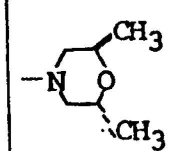 | oil | cis Ar/$CH_2$ | |
| 19 | 4-t-$C_4H_9$ | H | H | H | H | H | $CH_3$ | H | 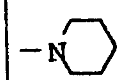 | oil | trans Ar/$CH_2$ | |
| 20 | 4-t-$C_4H_9$ | H | H | H | H | H | $CH_3$ | H | 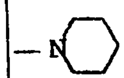 | oil | cis Ar/$CH_2$ | |

TABLE I (cont)

| Compound No. | X | Y | $R^1$ | $R^2$ | $R^3$ | $R^4$ | $R^5$ | $R^6$ | 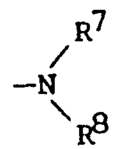 | m.p/b.p (°C) | Comments | $^1H$ nmr shifts (ppm from TMS) |
|---|---|---|---|---|---|---|---|---|---|---|---|---|
| 21 | 4-t-$C_4H_9$ | H | H | H | H | H | $CH_3$ | H | 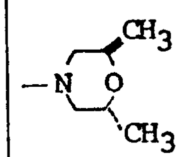 | oil | trans Ar/$CH_2$ | |
| 22 | 4-t-$C_4H_9$ | H | H | H | H | H | $CH_3$ | H | 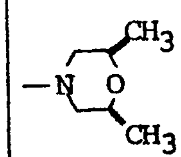 | oil | trans Ar/$CH_2$ | |
| 23 | 4-t-$C_4H_9$ | H | H | H | H | H | $CH_3$ | H | 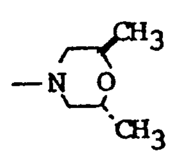 | oil | cis Ar/$CH_2$ | |
| 24 | 4-t-$C_4H_9$ | H | H | H | H | H | $CH_3$ | H | 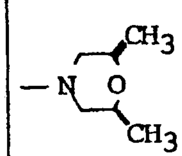 | oil | cis Ar/$CH_2$ | |

TABLE I (cont)

| Compound No. | X | Y | $R^1$ | $R^2$ | $R^3$ | $R^4$ | $R^5$ | $R^6$ | 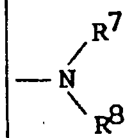 | m.p/b.p (°C) | Comments | $^1H$ nmr shifts (ppm from TMS) |
|---|---|---|---|---|---|---|---|---|---|---|---|---|
| 25 | 4-i-$C_3H_7$ | H | H | H | H | H | H | H | 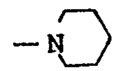 | oil | cis:trans Ar/$CH_2$ 2:3 | 2.1-2.5,4Hm* |
| 26 | 4-i-$C_3H_7$ | H | H | H | H | H | H | H | 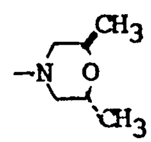 | oil | trans Ar/$CH_2$ | 3.8-4.2,2Hm$^+$ |
| 27 | 4-i-$C_3H_7$ | H | H | H | H | H | H | H | 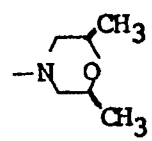 | oil | trans Ar/$CH_2$ | 3.5-3.9,2Hm$^+$ |
| 28 | 4-i-$C_3H_7$ | H | H | H | H | H | H | H | 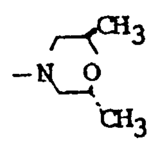 | oil | cis Ar/$CH_2$ | 3.5-4.2,2Hm$^+$ |
| 29 | 4-i-$C_3H_7$ | H | H | H | H | H | H | H | 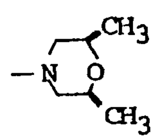 | oil | cis Ar/$CH_2$ | 3.4-3.8,2Hm$^+$ |

TABLE I (cont)

| Compound No. | X | Y | $R^1$ | $R^2$ | $R^3$ | $R^4$ | $R^5$ | $R^6$ | 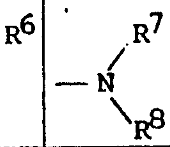 | m.p/b.p (°C) | Comments | $^1H$ nmr shifts (ppm from TMS) |
|---|---|---|---|---|---|---|---|---|---|---|---|---|
| 30 | 4-t-$C_4H_9$ | H | H | H | H | H | H | H | 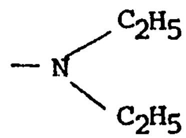 | oil | cis:trans Ar/$CH_2$=2:3 | |
| 31 | 4-t-$C_4H_9$ | H | Cl | Cl | H | H | H | H | 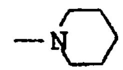 | oil | trans Ar/$CH_2$ | 2.3-2.6,4Hm* |
| 32 | 4-t-$C_4H_9$ | H | Cl | Cl | H | H | H | H | 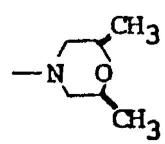 | oil | trans Ar/$CH_2$ | 3.6-3.8,2Hm$^+$ |
| 33 | 4-t-$C_4H_9$ | H | Cl | Cl | H | H | H | H | 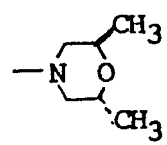 | oil | trans Ar/$CH_2$ | |
| 34 | 4-t-$C_4H_9$ | H | $CH_3$ | H | H | H | H | H | 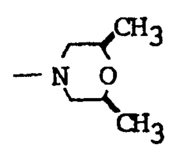 | oil | trans Ar/$CH_3$ trans Ar/$CH_2$ | 3.6-3.7,2Hm$^+$ |

TABLE I (cont)

| Compound No. | X | Y | $R^1$ | $R^2$ | $R^3$ | $R^4$ | $R^5$ | $R^6$ | 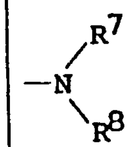 | m.p/b.p (°C) | Comments | $^1H$ nmr shifts (ppm from TMS) |
|---|---|---|---|---|---|---|---|---|---|---|---|---|
| 35 | 4-t-$C_4H_9$ | H | $CH_3$ | H | H | H | H | H | 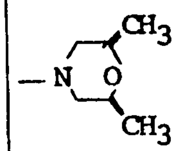 | oil | trans Ar/$CH_3$ cis Ar/$CH_2$ | 3.6-3.7,2Hm+ |
| 36 | 4-t-$C_4H_9$ | H | $CH_3$ | H | H | H | H | H | 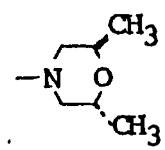 | oil | trans Ar/$CH_3$ trans Ar/$CH_2$ | 3.8-4.2,2Hm+ |
| 37 | 4-t-$C_4H_9$ | H | $CH_3$ | H | H | H | H | H | 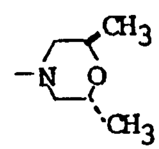 | oil | trans Ar/$CH_3$ cis Ar/$CH_2$ | |
| 38 | 4-t-$C_4H_9O$ | H | H | H | H | H | H | H | 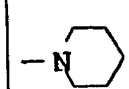 | oil | cis : trans Ar/$CH_2$ 3:7 | 2.1-2.6,4Hm* |

TABLE I (cont)

| Compound No. | X | Y | $R^1$ | $R^2$ | $R^3$ | $R^4$ | $R^5$ | $R^6$ | 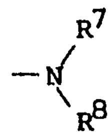 | m.p/b.p (°C) | Comments | $^1H$ nmr shifts (ppm from TMS) |
|---|---|---|---|---|---|---|---|---|---|---|---|---|
| 39 | 4-t-$C_4H_9O$ | H | H | H | H | H | H | H | 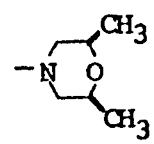 | oil | trans Ar/$CH_2$ | 4.5-4.9,2Hm+ |
| 40 | 4-t-$C_4H_9O$ | H | H | H | H | H | H | H | 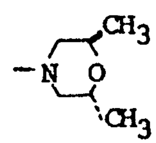 | oil | trans Ar/$CH_2$ | 4.8-5.2,2Hm+ |
| 41 | 4-$CH_3$ | H | H | H | H | H | H | H | 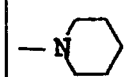 | oil | cis : trans AR/$CH_2$ 1:2 | 2.2-2.6,4Hm* |
| 42 | 4-$CH_3$ | H | H | H | H | H | H | H | 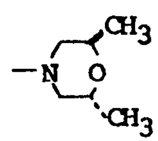 | oil | trans Ar/$CH_2$ | 3.8-4.2,2Hm+ |
| 43 | 4-$CH_3$ | H | H | H | H | H | H | H | 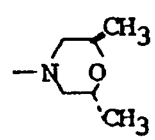 | oil | cis : trans AR/$CH_2$ 3:2 | 3.5-4.2,2Hm+ |

TABLE I (cont)

| Compound No. | X | Y | $R^1$ | $R^2$ | $R^3$ | $R^4$ | $R^5$ | $R^6$ | 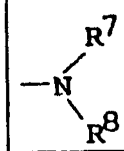 | m.p/b.p (°C) | Comments | $^1H$ nmr shifts (ppm from TMS) |
|---|---|---|---|---|---|---|---|---|---|---|---|---|
| 44 | 4-$CH_3$ | H | H | H | H | H | H | H | 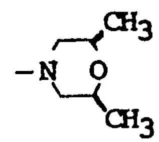 | oil | trans Ar/$CH_2$ | 3.5-3.9,2Hm$^+$ |
| 45 | 4-$CH_3$ | H | H | H | H | H | H | H | 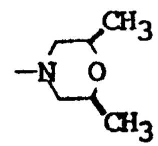 | oil | cis Ar/$CH_2$ | 3.5-3.8,2Hm$^+$ |
| 46 | 4-Br | H | H | H | H | H | H | H | 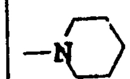 | oil | cis : trans AR/$CH_2$ 1:1 | 2.1-2.6,4Hm* |
| 47 | 4-Br | H | H | H | H | H | H | H | 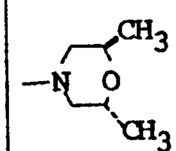 | oil | cis : trans AR/$CH_2$ 2:3 | 3.8-4.2,2Hm$^+$ |
| 48 | 4-Br | H | H | H | H | H | H | H | 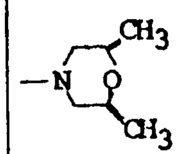 | oil | trans Ar/$CH_2$ | 3.4-3.9,2Hm$^+$ |

TABLE I (cont)

| Compound No. | X | Y | $R^1$ | $R^2$ | $R^3$ | $R^4$ | $R^5$ | $R^6$ | 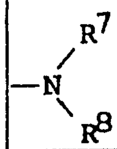 | m.p/b.p (°C) | Comments | $^1H$ nmr shifts (ppm from TMS) |
|---|---|---|---|---|---|---|---|---|---|---|---|---|
| 49 | 4-Br | H | H | H | H | H | H | H | 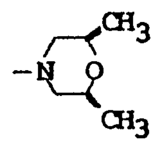 | oil | cis $Ar/CH_2$ | 3.4-3.8, 2Hm[+] |
| 50 | $3-OC_6H_5$ | H | H | H | H | H | H | H | 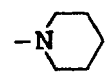 | oil | cis : trans $Ar/CH_2$ 2:3 | 2.1-2.6, 4Hm* |
| 51 | $3-OC_6H_5$ | H | H | H | H | H | H | H | 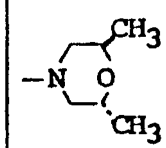 | oil | cis : trans $Ar/CH_2$ 2:3 | 3.8-4.2, 2Hm[+] |
| 52 | $3-OC_6H_5$ | H | H | H | H | H | H | H | 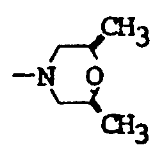 | oil | trans $Ar/CH_2$ | 3.5-3.9, 2Hm[+] |
| 53 | $3-OC_6H_5$ | H | H | H | H | H | H | H | 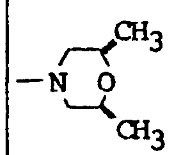 | oil | cis $Ar/CH_2$ | 3.4-3.8, 2Hm[+] |

TABLE I (cont)

| Compound No. | X | Y | $R^1$ | $R^2$ | $R^3$ | $R^4$ | $R^5$ | $R^6$ | 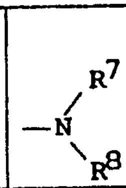 | m.p/b.p (°C) | Comments | $^1H$ nmr shifts (ppm from TMS) |
|---|---|---|---|---|---|---|---|---|---|---|---|---|
| 54 | H | H | H | H | H | H | H | H | 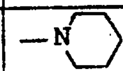 | oil | cis : trans Ar/$CH_2$ 1:2 | 2.0-2.6,4Hm* |
| 55 | H | H | H | H | H | H | H | H | 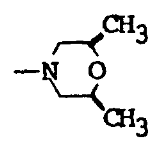 | oil | trans Ar/$CH_2$ | 3.5-3.8,2Hm+ |
| 56 | H | H | H | H | H | H | H | H | 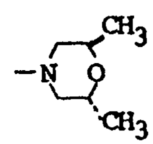 | oil | cis : trans Ar/$CH_2$ 2:3 | 3.8-4.1,2Hm+ |
| 57 | H | H | H | H | H | H | H | H | 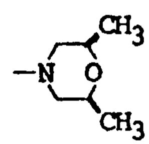 | oil | cis Ar/$CH_2$ | 3.4-3.8,2Hm+ |
| 58 | 4-t-$C_4H_9$ | H | $CH_3$ | $CH_3$ | H | H | H | H | 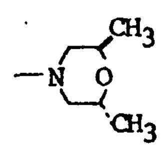 | oil | trans Ar/$CH_2$ | 3.9-4.2,2Hm+ |

TABLE I (cont)

| Compound No. | X | Y | $R^1$ | $R^2$ | $R^3$ | $R^4$ | $R^5$ | $R^6$ | 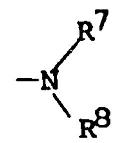 | m.p/b.p (°C) | Comments | $^1H$ nmr shifts (ppm from TMS) |
|---|---|---|---|---|---|---|---|---|---|---|---|---|
| 59 | 4-t-$C_4H_9$ | H | $CH_3$ | $CH_3$ | H | H | H | H | 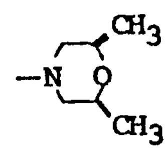 | oil | trans Ar/$CH_2$ | 3.6-3.8, 2Hm+ |
| 60 | 3-$OCH_2C_6H_5$ | H | H | H | H | H | H | H | 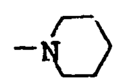 | oil | cis:trans Ar/$CH_2$ 2:3 | 2.2-2.6, 4Hm* |
| 61 | 3-$OCH_2C_6H_5$ | H | H | H | H | H | H | H | 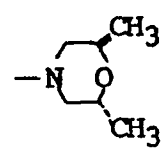 | oil | cis : trans Ar/$CH_2$ 3:2 | 3.9-4.1, 2Hm+ |
| 62 | 3-$OCH_2C_6H_5$ | H | H | H | H | H | H | H | 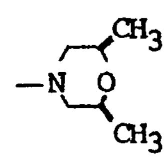 | oil | trans Ar/$CH_2$ | 3.6-3.7, 2Hm+ |

TABLE I (cont)

| Compound No. | X | Y | $R^1$ | $R^2$ | $R^3$ | $R^4$ | $R^5$ | $R^6$ | 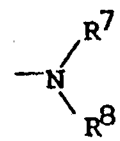 | m.p/b.p (°C) | Comments | $^1H$ nmr shifts (ppm from TMS) |
|---|---|---|---|---|---|---|---|---|---|---|---|---|
| 63 | $3\text{-}OCH_2C_6H_5$ | H | H | H | H | H | H | H | 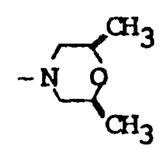 | oil | cis $Ar/CH_2$ | 3.5–3.7,2Hm$^+$ |
| 64 | 4-Cl | H | H | H | H | H | H | H | 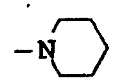 | oil | cis : trans $Ar/CH_2$ 1:2 | 2.0–2.6,4Hm* |
| 65 | 4-Cl | H | H | H | H | H | H | H | 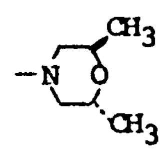 | oil | cis : trans $Ar/CH_2$ 1:1 | 3.8–4.2,2Hm$^+$ |
| 66 | 4-Cl | H | H | H | H | H | H | H | 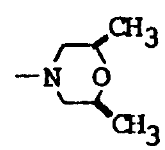 | oil | trans $Ar/CH_2$ | 3.5–3.9,2Hm$^+$ |
| 67 | 4-Cl | H | H | H | H | H | H | H | 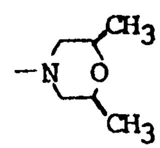 | oil | cis $Ar/CH_2$ | 3.4–3.8,2Hm$^+$ |

TABLE I (cont)

| Compound No. | X | Y | $R^1$ | $R^2$ | $R^3$ | $R^4$ | $R^5$ | $R^6$ | 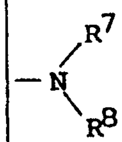 | m.p/b.p (°C) | Comments | $^1H$ nmr shifts (ppm from TMS) |
|---|---|---|---|---|---|---|---|---|---|---|---|---|
| 68 | 3-$OCH_3$ | H | H | H | H | H | H | H | 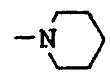 | oil | cis : trans Ar/$CH_2$ 3:7 | 2.1-2.6,4Hm* |
| 69 | 3-$OCH_3$ | H | H | H | H | H | H | H | 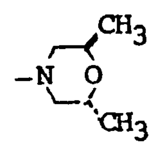 | oil | cis : trans Ar/$CH_2$ 1:2 | 3.9-4.1,2Hm+ |
| 70 | 3-$OCH_3$ | H | H | H | H | H | H | H | 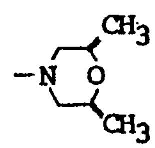 | oil | trans Ar/$CH_2$ | 3.6-3.8,2Hm+ |
| 71 | 3-$OCH_3$ | H | H | H | H | H | H | H | 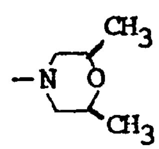 | oil | cis Ar/$CH_2$ | 3.6-3.8,2Hm+ |
| 72 | 4-$OC_6H_5$ | H | H | H | H | H | H | H | 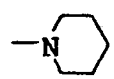 | oil | cis : trans Ar/$CH_2$ 1:1 | 2.1-2.5,4Hm* |

TABLE I (cont)

| Compound No. | X | Y | $R^1$ | $R^2$ | $R^3$ | $R^4$ | $R^5$ | $R^6$ | 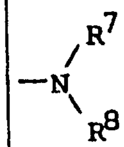 | m.p/b.p (°C) | Comments | $^1H$ nmr shifts (ppm from TMS) |
|---|---|---|---|---|---|---|---|---|---|---|---|---|
| 73 | 4-$OC_6H_5$ | H | H | H | H | H | H | H | 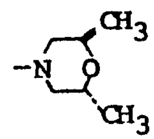 | oil | cis : trans Ar/$CH_2$ 2:3 | 3.9–4.1, 2Hm$^+$ |
| 74 | 4-$OC_6H_5$ | H | H | H | H | H | H | H | 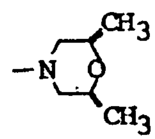 | oil | trans Ar/$CH_2$ | 3.6–3.8, 2Hm$^+$ |
| 75 | 4-$OC_6H_5$ | H | H | H | H | H | H | H | 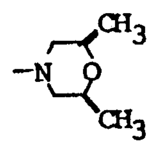 | oil | cis Ar/$CH_2$ | 3.6–3.8, 2Hm$^+$ |
| 76 | 4-$C_6H_5$ | H | H | H | H | H | H | H | 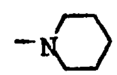 | oil | cis : trans Ar/$CH_2$ 2:3 | 2.3–2.6, 4Hm* |
| 77 | 4-$C_6H_5$ | H | H | H | H | H | H | H | 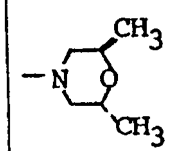 | oil | cis : trans Ar/$CH_2$ 1:2 | 3.9–4.1, 2Hm$^+$ |

TABLE I (cont)

| Compound No. | X | Y | $R^1$ | $R^2$ | $R^3$ | $R^4$ | $R^5$ | $R^6$ | 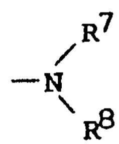 | m.p/b.p (°C) | Comments | $^1H$ nmr shifts (ppm from TMS) |
|---|---|---|---|---|---|---|---|---|---|---|---|---|
| 78 | $4-C_6H_5$ | H | H | H | H | H | H | H | 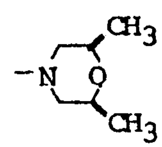 | oil | trans $Ar/CH_2$ | 3.6-3.8, 2Hm$^+$ |
| 79 | $4-C_6H_5$ | H | H | H | H | H | H | H | 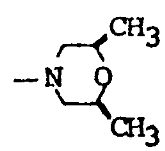 | oil | cis $Ar/CH_2$ | 3.5-3.7, 2Hm$^+$ |
| 80 | $3-CH_3$ | H | H | H | H | H | H | H | 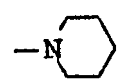 | oil | cis : trans $Ar/CH_2$ 1:2 | 3.4-3.8, 4Hm* |
| 81 | $3-CH_3$ | H | H | H | H | H | H | H | 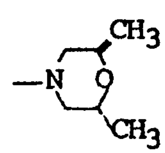 | oil | trans $Ar/CH_2$ | 3.8-4.1, 2Hm$^+$ |

TABLE I (cont)

| Compound No. | X | Y | $R^1$ | $R^2$ | $R^3$ | $R^4$ | $R^5$ | $R^6$ | 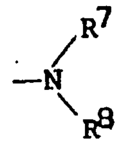 | m.p/b.p (°C) | Comments | $^1H$ nmr shifts (ppm from TMS) |
|---|---|---|---|---|---|---|---|---|---|---|---|---|
| 82 | 3-$CH_3$ | H | H | H | H | H | H | H | 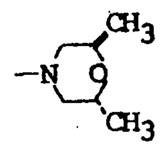 | oil | cis :trans Ar/$CH_2$ 2:3 | 3.9-4.1,2Hm+ |
| 83 | 3-$CH_3$ | H | H | H | H | H | H | H | 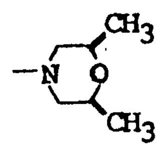 | oil | trans Ar/$CH_2$ | 3.6-3.8,2Hm+ |
| 84 | 3-$CH_3$ | H | H | H | H | H | H | H | 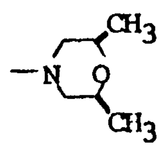 | oil | cis Ar/$CH_2$ | 3.4-3.8,2Hm+ |
| 85 | 4-$OCH_3$ | H | H | H | H | H | H | H | 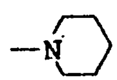 | oil | cis:trans Ar/$CH_2$=3:7 | 2.3-2.6,4Hm* |

TABLE I (cont)

| Compound No. | X | Y | $R^1$ | $R^2$ | $R^3$ | $R^4$ | $R^5$ | $R^6$ | 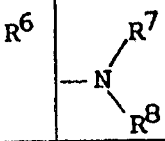 | m.p/b.p (°C) | Comments | $^1H$ nmr shifts (ppm from TMS) |
|---|---|---|---|---|---|---|---|---|---|---|---|---|
| 86 | 4-$OCH_3$ | H | H | H | H | H | H | H | 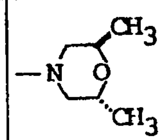 | oil | cis:trans Ar/$CH_2$=1:2 | 3.9-4.1, 2Hm[+] |
| 87 | 4-$OCH_3$ | H | H | H | H | H | H | H | 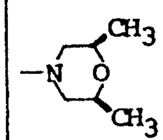 | oil | trans Ar/$CH_2$ | 3.6-3.7, 2Hm[+] |
| 88 | 4-$OCH_3$ | H | H | H | H | H | H | H | 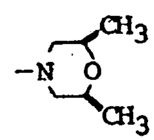 | oil | cis Ar/$CH_2$ | 3.6-3.7, 2Hm[+] |
| 89 | 4-$OCH_3$ | H | H | H | H | H | H | H | 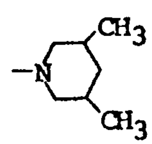 | oil | cis:trans $CH_3$/$CH_3$=1:1<br>cis:trans Ar/$CH_2$=1:2 | 2.1-2.5, 4Hm[+] |

TABLE I (cont)

| Compound No. | X | Y | $R^1$ | $R^2$ | $R^3$ | $R^4$ | $R^5$ | $R^6$ | 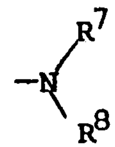 | m.p/b.p (°C) | Comments | $^1H$ nmr shifts (ppm from TMS) |
|---|---|---|---|---|---|---|---|---|---|---|---|---|
| 90 | 4-$OCH_3$ | H | H | H | H | H | H | H | 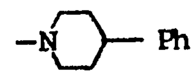 | 67-68 | cis Ar/$CH_2$ | 2.1-2.6, 4Hm* |
| 91 | 4-$OCH_3$ | H | H | H | H | H | H | H | 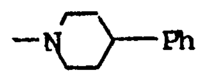 | oil | trans Ar/$CH_2$ | 2.1-2.5, 4Hm* |
| 92 | 4-i-$C_3H_7$ | H | $CH_3$ | H | H | H | H | H | 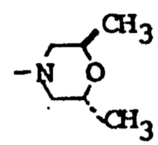 | oil | trans Ar/$CH_3$<br>trans Ar/$CH_2$ | 3.9-4.1, 2Hm+ |
| 93 | 4-i-$C_3H_7$ | H | $CH_3$ | H | H | H | H | H | 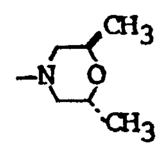 | oil | trans Ar/$CH_3$<br>cis Ar/$CH_2$ | 3.5-3.7, 2Hm+ |
| 94 | 4-i-$C_3H_7$ | H | $CH_3$ | H | H | H | H | H | 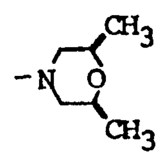 | oil | trans Ar/$CH_3$<br>trans Ar/$CH_2$ | 3.5-3.8, 2Hm+ |

TABLE I (cont)

| Compound No. | X | Y | $R^1$ | $R^2$ | $R^3$ | $R^4$ | $R^5$ | $R^6$ | 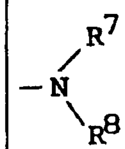 | m.p/b.p (°C) | Comments | $^1H$ nmr shifts (ppm from TMS) |
|---|---|---|---|---|---|---|---|---|---|---|---|---|
| 95 | 4-<u>i</u>-$C_3H_7$ | H | $CH_3$ | H | H | H | H | H | 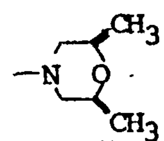 | oil | trans Ar/$CH_3$ cis Ar/$CH_2$ | 3.5-3.7,2Hm$^+$ |
| 96 | 3-Br | H | H | H | H | H | H | H | 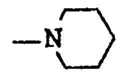 | oil | cis:trans Ar/$CH_2$=1:1 | 2.2-2.5,4Hm* |
| 97 | 3-Br | H | H | H | H | H | H | H | 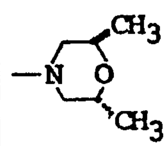 | oil | trans Ar/$CH_2$ | 3.9-4.1,2Hm$^+$ |
| 98 | 3-Br | H | H | H | H | H | H | H | 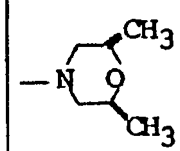 | oil | trans Ar/$CH_2$ | 3.6-3.8,2Hm$^+$ |

TABLE I (cont)

| Compound No. | X | Y | $R^1$ | $R^2$ | $R^3$ | $R^4$ | $R^5$ | $R^6$ | 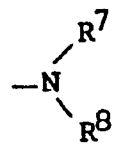 | m.p/b.p (°C) | Comments | $^1H$ nmr shifts (ppm from TMS) |
|---|---|---|---|---|---|---|---|---|---|---|---|---|
| 99 | 3-Br | H | H | H | H | H | H | H | 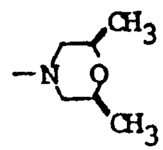 | oil | cis Ar/$CH_2$ | 3.6-3.7,2Hm+ |
| 100 | 4-t-$C_4H_9$ | H | H | H | H | H | H | H | 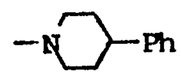 | oil | trans Ar/$CH_2$ | 1.3,9Hs (t-$C_4H_9$) |
| 101 | 4-t-$C_4H_9$ | H | H | H | H | H | H | H | 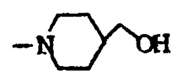 | oil | trans Ar/$CH_2$ | 1.25,9Hs (t-$C_4H_9$) |
| 102 | 4-$(CH_3)_3Si$ | H | H | H | H | H | H | H | 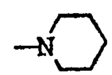 | oil | trans Ar/$CH_2$ | 2.2-2.5,4Hm* |
| 103 | 4-$(CH_3)_3Si$ | H | H | H | H | H | H | H | 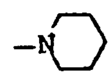 | oil | cis Ar/$CH_2$ | 2.0-2.4,4Hm* |

TABLE I (cont)

| Compound No. | X | Y | $R^1$ | $R^2$ | $R^3$ | $R^4$ | $R^5$ | $R^6$ | 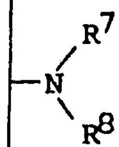 | m.p/b.p (°C) | Comments | $^1H$ nmr shifts (ppm from TMS) |
|---|---|---|---|---|---|---|---|---|---|---|---|---|
| 104 | 3-$(CH_3)_3Si$ | H | H | H | H | H | H | H | 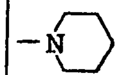 | oil | trans Ar/$CH_2$ | 2.2-2.5,4Hm* |
| 105 | 3-$(CH_3)_3Si$ | H | H | H | H | H | H | H | 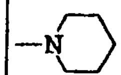 | oil | cis Ar/$CH_2$ | 2.1-2.4,4Hm* |
| 106 | 4-t-$C_4H_9$ | H | H | H | H | H | H | H | 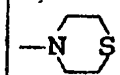 | oil | trans Ar/$CH_2$ | 1.3,9Hs (t-$C_4H_9$) |
| 107 | 4-t-$C_4H_9$ | H | H | H | H | H | H | H | 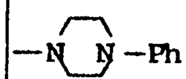 | oil | cis Ar/$CH_2$ | 1.35,9Hs (t-$C_4H_9$) |
| 108 | 4-t-$C_4H_9$ | H | H | H | H | H | H | H | 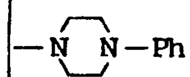 | oil | trans Ar/$CH_2$ | 1.3,9Hs (t-$C_4H_9$) |
| 109 | 4-t-$C_4H_9$ | H | $CH_3$ | H | H | H | H | $CH_3$ | 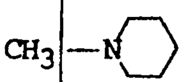 | oil | trans Ar/$CH_3$ cis Ar/$CH_2$ | 2.1-2.6,4Hm* |

TABLE I (cont)

| Compound No. | X | Y | $R^1$ | $R^2$ | $R^3$ | $R^4$ | $R^5$ | $R^6$ | 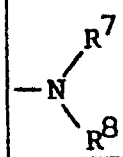 | m.p/b.p (°C) | Comments | $^1H$ nmr shifts (ppm from TMS) |
|---|---|---|---|---|---|---|---|---|---|---|---|---|
| 110 | 4-t-$C_4H_9$ | H | $CH_3$ | H | H | H | H | H | 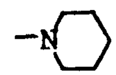 | oil | cis Ar/$CH_3$ trans Ar/$CH_2$ | 2.2-2.8,4Hm* |
| 111 | 4-t-$C_4H_9$ | H | $CH_3$ | H | H | H | H | $CH_3$ | 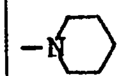 | oil | trans Ar/$CH_3$ trans Ar/$CH_2$ | 2.2-2.8,4Hm* |
| 112 | 4-i-$C_3H_7$ | H | $CH_3$ | H | H | H | H | H | 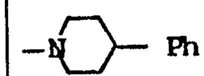 | oil | trans Ar/$CH_3$ cis Ar/$CH_2$ | 2.25,6Hd (i-$C_3H_7$) |
| 113 | 4-i-$C_3H_7$ | H | $CH_3$ | H | H | H | H | H | 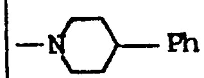 | oil | trans Ar/$CH_3$ trans Ar/$CH_2$ | 2.25,6Hd (i-$C_3H_7$) |
| 114 | 4-i-$C_3H_7$ | H | $CH_3$ | H | H | H | H | H | 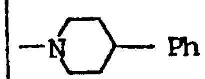 | oil | cis Ar/$CH_3$ trans Ar/$CH_3$ | 2.25,6Hd (i-$C_3H_7$) |

TABLE I (cont)

| Compound No. | X | Y | $R^1$ | $R^2$ | $R^3$ | $R^4$ | $R^5$ | $R^6$ | 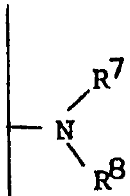 | m.p/b.p (°C) | Comments | $^1H$ nmr shifts (ppm from TMS) |
|---|---|---|---|---|---|---|---|---|---|---|---|---|
| 115 | 4-i-$C_3H_7$ | H | $CH_3$ | H | H | H | H | H | 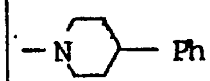 | oil | cis Ar/$CH_3$ cis Ar/$CH_2$ | 2.25,6Hd (i-$C_3H_7$) |
| 116 | 4-t-$C_4H_9$ | H | $CH_3$ | H | H | H | H | $CH_3$ | 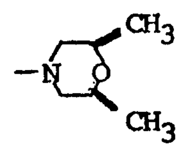 | oil | trans Ar/$CH_3$ cis Ar/$CH_2$ | 3.6-3.8,2Hm+ |
| 117 | 4-t-$C_4H_9$ | H | $CH_3$ | H | H | H | H | $CH_3$ | 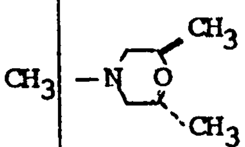 | oil | cis Ar/$CH_3$ trans Ar/$CH_2$ | 3.9-4.1,2Hm+ |
| 118 | 4-t-$C_4H_9$ | H | $CH_3$ | H | H | H | H | $CH_3$ | 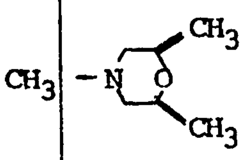 | oil | cis Ar/$CH_3$ trans Ar/$CH_2$ | 3.6-3.8,2Hm+ |

TABLE I (cont)

| Compound No. | X | Y | $R^1$ | $R^2$ | $R^3$ | $R^4$ | $R^5$ | $R^6$ | 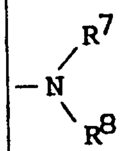 | m.p/b.p (°C) | Comments | $^1H$ nmr shifts (ppm from TMS) |
|---|---|---|---|---|---|---|---|---|---|---|---|---|
| 119 | 4-t-$C_4H_9$ | H | $CH_3$ | H | H | H | H | $CH_3$ | 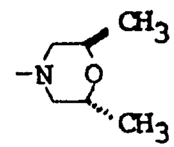 | oil | trans Ar/$CH_3$ trans Ar/$CH_2$ | 3.9-4.1, 2Hm+ |
| 120 | 4-t-$C_4H_9$ | H | $CH_3$ | H | H | H | H | $CH_3$ | 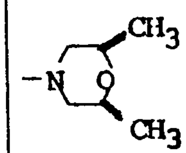 | oil | trans Ar/$CH_3$ trans Ar/$CH_2$ | 3.6-3.7, 2Hm+ |
| 121 | 4-i-$C_3H_7$ | H | H | H | H | H | H | $CH_3$ | 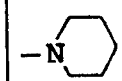 | oil | cis:trans Ar/$CH_2$=1:2 | |
| 122 | 4-i-$C_3H_7$ | H | H | H | H | H | H | $CH_3$ | 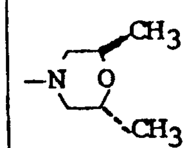 | oil | cis:trans Ar/$CH_2$=1:1 | |

TABLE I (cont)

| Compound No. | X | Y | $R^1$ | $R^2$ | $R^3$ | $R^4$ | $R^5$ | $R^6$ | 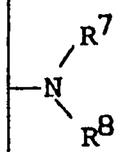 | m.p/b.p (°C) | Comments | $^1H$ nmr shifts (ppm from TMS) |
|---|---|---|---|---|---|---|---|---|---|---|---|---|
| 123 | 4-i-$C_3H_7$ | H | H | H | H | H | H | $CH_3$ | 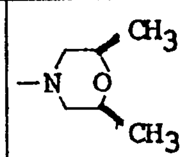 | oil | trans Ar/$CH_2$ | |
| 124 | 4-i-$C_3H_7$ | H | H | H | H | H | H | $CH_3$ | 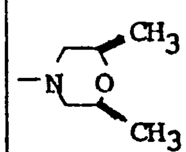 | oil | cis Ar/$CH_2$ | |
| 125 | 3-t-$C_4H_9$ | H | H | H | H | H | H | H | 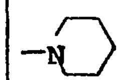 | oil | cis:trans Ar/$CH_2$=1:2 | |
| 126 | 3-t-$C_4H_9$ | H | H | H | H | H | H | H | 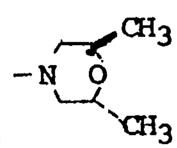 | oil | cis:trans Ar/$CH_2$=1:1 | |

TABLE I (cont)

| Compound No. | X | Y | $R^1$ | $R^2$ | $R^3$ | $R^4$ | $R^5$ | $R^6$ | 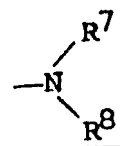 | m.p/b.p (°C) | Comments | $^1H$ nmr shifts (ppm from TMS) |
|---|---|---|---|---|---|---|---|---|---|---|---|---|
| 127 | 3-t-$C_4H_9$ | H | H | H | H | H | H | H | 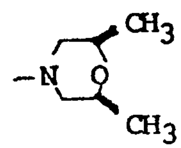 | oil | trans Ar/$CH_2$ | |
| 128 | 3-t-$C_4H_9$ | H | H | H | H | H | H | H | 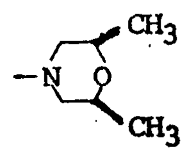 | oil | cis Ar/$CH_2$ | |
| 129 | 3-$(CH_3)_3Si$ | H | H | H | H | H | H | H | 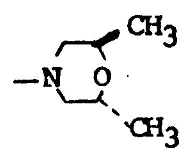 | oil | cis:trans Ar/$CH_2$=1:1 | 3.9-4.1, 2Hm$^+$ |
| 130 | 3-$(CH_3)_3Si$ | H | H | H | H | H | H | H | 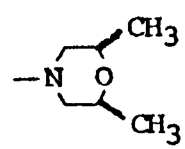 | oil | trans Ar/$CH_2$ | 3.6-3.8, 2Hm$^+$ |

TABLE I (cont)

| Compound No. | X | Y | $R^1$ | $R^2$ | $R^3$ | $R^4$ | $R^5$ | $R^6$ | 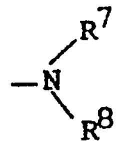 | m.p/b.p (°C) | Comments | $^1H$ nmr shifts (ppm from TMS) |
|---|---|---|---|---|---|---|---|---|---|---|---|---|
| 131 | 3-$(CH_3)_3Si$ | H | H | H | H | H | H | H | 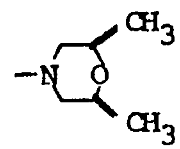 | oil | cis $Ar/CH_2$ | 3.5-3.7, $2Hm^+$ |
| 132 | 4-$(CH_3)_3Si$ | H | H | H | H | H | H | H | 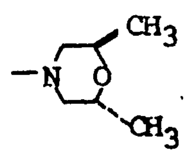 | oil | cis:trans $Ar/CH_2$=1:1 | 3.9-4.1, $2Hm^+$ |
| 133 | 4-$(CH_3)_3Si$ | H | H | H | H | H | H | H | 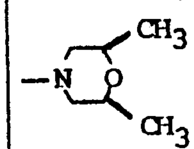 | oil | trans $Ar/CH_2$ | 3.5-3.8, $2Hm^+$ |
| 134 | 4-$(CH_3)_3Si$ | H | H | H | H | H | H | H | 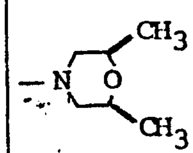 | oil | cis $Ar/CH_2$ | 3.5-3.7, $2Hm^+$ |

TABLE I (cont)

| Compound No. | X | Y | $R^1$ | $R^2$ | $R^3$ | $R^4$ | $R^5$ | $R^6$ | 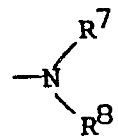 | m.p/b.p (°C) | Comments | $^1H$ nmr shifts (ppm from TMS) |
|---|---|---|---|---|---|---|---|---|---|---|---|---|
| 135 | 4-n-$C_4H_9$ | H | H | H | H | H | H | H | 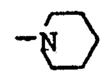 | | trans Ar/$CH_2$ | |
| 136 | 4-n-$C_4H_9$ | H | H | H | H | H | H | H | 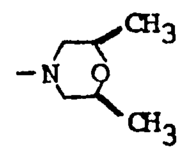 | | trans Ar/$CH_2$ | |
| 137 | 4-n-$C_4H_9$ | H | H | H | H | H | H | H | 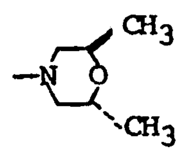 | | trans Ar/$CH_2$ | |
| 138 | 4-s-$C_4H_9$ | H | H | H | H | H | H | H | 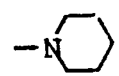 | | trans Ar/$CH_2$ | |
| 139 | 4-s-$C_4H_9$ | H | H | H | H | H | H | H | 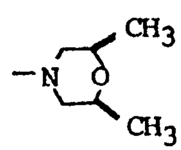 | | trans Ar/$CH_2$ | |

TABLE I (cont)

| Compound No. | X | Y | $R^1$ | $R^2$ | $R^3$ | $R^4$ | $R^5$ | $R^6$ | 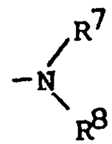 | m.p/b.p (°C) | Comments | $^1H$ nmr shifts (ppm from TMS) |
|---|---|---|---|---|---|---|---|---|---|---|---|---|
| 140 | 4-s-$C_4H_9$ | H | H | H | H | H | H | H | 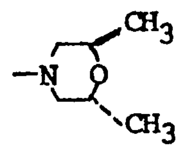 | | trans Ar/$CH_2$ | |
| 141 | 4-n-$C_3H_7$ | H | H | H | H | H | H | H | 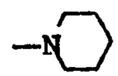 | | trans Ar/$CH_2$ | |
| 142 | 4-n-$C_3H_7$ | H | H | H | H | H | H | H | 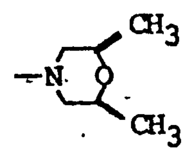 | | trans Ar/$CH_2$ | |
| 143 | 4-n-$C_3H_7$ | H | H | H | H | H | H | H | 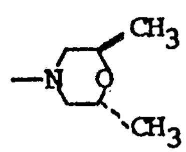 | | trans Ar/$CH_2$ | |
| 144 | 4-i-$C_3H_7$ | 2-$CH_3$ | H | H | H | H | H | H | 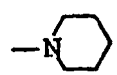 | | trans Ar/$CH_2$ | |

TABLE I (cont)

| Compound No. | X | Y | $R^1$ | $R^2$ | $R^3$ | $R^4$ | $R^5$ | $R^6$ | 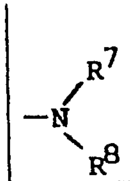 | m.p/b.p (°C) | Comments | $^1H$ nmr shifts (ppm from TMS) |
|---|---|---|---|---|---|---|---|---|---|---|---|---|
| 145 | 4-i-$C_3H_7$ | 2-$CH_3$ | H | H | H | H | H | H | 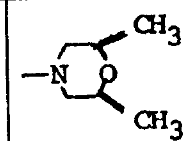 | | trans Ar/$CH_2$ | |
| 146 | 4-i-$C_3H_7$ | 2-$CH_3$ | H | H | H | H | H | H | 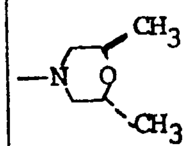 | | trans Ar/$CH_2$ | |
| 147 | 4-$(CH_3)_3Si$ | H | H | H | H | H | H | $CH_3$ | 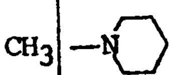 | | trans Ar/$CH_2$ | |
| 148 | 4-$(CH_3)_3Si$ | H | H | H | H | H | H | $CH_3$ | 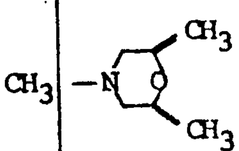 | | trans Ar/$CH_2$ | |
| 149 | 4-$(CH_3)_3Si$ | H | H | H | H | H | H | $CH_3$ | 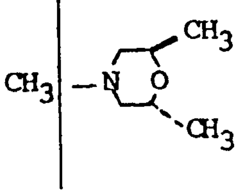 | | trans Ar/$CH_2$ | |

TABLE I (cont)

| Compound No. | X | Y | $R^1$ | $R^2$ | $R^3$ | $R^4$ | $R^5$ | $R^6$ | 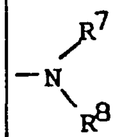 | m.p/b.p (°C) | Comments | $^1H$ nmr shifts (ppm from TMS) |
|---|---|---|---|---|---|---|---|---|---|---|---|---|
| 150 | 4-$(CH_3)_3Si$ | H | $CH_3$ | H | H | H | H | H | 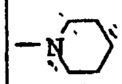 | | trans Ar/$CH_3$ trans Ar/$CH_2$ | |
| 151 | 4-$(CH_3)_3Si$ | H | $CH_3$ | H | H | H | H | H | 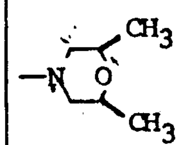 | | trans Ar/$CH_3$ trans Ar/$CH_2$ | |
| 152 | 4-$(CH_3)_3Si$ | H | $CH_3$ | H | H | H | H | H | 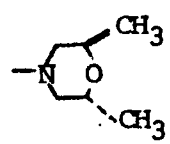 | | trans Ar/$CH_3$ trans Ar/$CH_2$ | |
| 153 | 4-t-$C_4H_9$ | H | H | H | $CH_3$ | H | H | H | 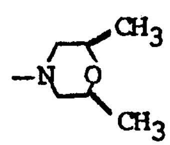 | | trans Ar/$CH(CH_3)$ | |
| 154 | 4-t-$C_4H_9$ | H | H | H | $CH_3$ | H | H | H | 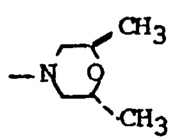 | | trans Ar/$CH(CH_3)$ | |

TABLE I (cont)

| Compound No. | X | Y | $R^1$ | $R^2$ | $R^3$ | $R^4$ | $R^5$ | $R^6$ | 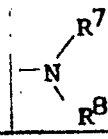 | m.p/b.p (°C) | Comments | $^1H$ nmr shifts (ppm from TMS) |
|---|---|---|---|---|---|---|---|---|---|---|---|---|
| 155 | 4-t-$C_4H_9$ | H | H | H | $CH_3$ | $CH_3$ | H | H | 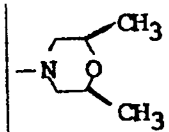 | | trans Ar/$C(CH_3)_2$ | |
| 156 | 4-t-$C_4H_9$ | H | H | H | $CH_3$ | $CH_3$ | H | H | 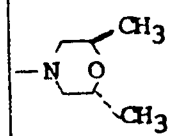 | | trans Ar/$C(CH_3)_2$ | |
| 157 | 4-t-$C_4H_9$ | H | n-$C_4H_9$ | H | H | H | H | H | 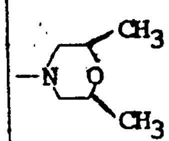 | | trans Ar/n-$C_4H_9$ trans Ar/$CH_2$ | |
| 158 | 4-t-$C_4H_9$ | H | n-$C_4H_9$ | H | H | H | H | H | 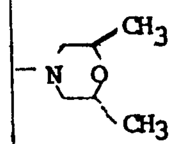 | | trans Ar/n-$C_4H_9$ trans Ar/$CH_2$ | |
| 159 | 4-t-$C_4H_9$ | H | n-$C_4H_9$ | n-$C_4H_9$ | H | H | H | H | 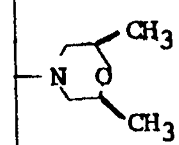 | | trans Ar/$CH_2$ | |

TABLE I (cont)

| Compound No. | X | Y | $R^1$ | $R^2$ | $R^3$ | $R^4$ | $R^5$ | $R^6$ | 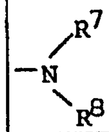 | m.p/b.p (°C) | Comments | $^1H$ nmr shifts (ppm from TMS) |
|---|---|---|---|---|---|---|---|---|---|---|---|---|
| 160 | 4-t-$C_4H_9$ | H | n-$C_4H_9$ | n-$C_4H_9$ | H | H | H | H | 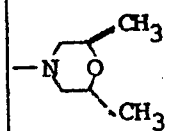 | | trans Ar/$CH_2$ | |
| 161 | 4-t-$C_4H_9$ | H | H | H | H | H | H | n-$C_4H_9$ | 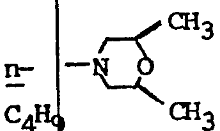 | | trans Ar/$CH_2$ | |
| 162 | 4-t-$C_4H_9$ | H | H | H | H | H | H | n-$C_4H_9$ | 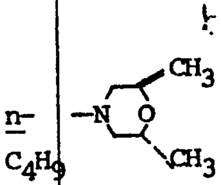 | | trans Ar/$CH_2$ | |
| 163 | 4-t-$C_4H_9$ | H | H | H | H | H | n-$C_4H_9$ | H | 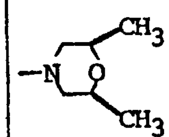 | | trans Ar/$CH_2$ | |
| 164 | 4-t-$C_4H_9$ | H | H | H | H | H | n-$C_4H_9$ | H | 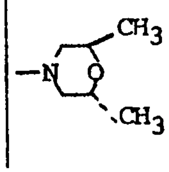 | | trans Ar/$CH_2$ | |

TABLE I (cont)

| Compound No. | X | Y | $R^1$ | $R^2$ | $R^3$ | $R^4$ | $R^5$ | $R^6$ | 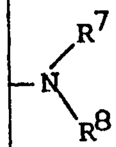 | m.p/b.p (°C) | Comments | $^1H$ nmr shifts (ppm from TMS) |
|---|---|---|---|---|---|---|---|---|---|---|---|---|
| 165 | 4-t-$C_4H_9$ | H | H | H | H | H | n-$C_4H_9$ | n-$C_4H_9$ | 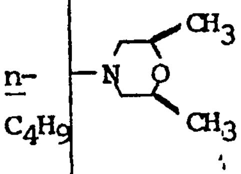 | | trans Ar/$CH_2$ | |
| 166 | 4-t-$C_4H_9$ | H | H | H | H | H | n-$C_4H_9$ | n-$C_4H_9$ | 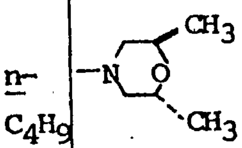 | | trans Ar/$CH_2$ | |
| 167 | 4-t-$C_4H_9$ | H | H | H | H | H | H | H | $-NH_2$ | | trans Ar/$CH_2$ | |
| 168 | 4-t-$C_4H_9$ | H | H | H | H | H | H | H | NHn-$C_4H_9$ | | trans Ar/$CH_2$ | |
| 169 | $C_6H_{11}$ cyclohexyl | H | H | H | H | H | H | H | 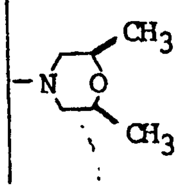 | | trans Ar/$CH_2$ | |

TABLE I (cont)

| Compound No. | X | Y | $R^1$ | $R^2$ | $R^3$ | $R^4$ | $R^5$ | $R^6$ | 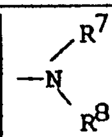 | m.p/b.p (°C) | Comments | $^1H$ nmr shifts (ppm from TMS) |
|---|---|---|---|---|---|---|---|---|---|---|---|---|
| 170 | $C_6H_{11}$ cyclohexyl | H | H | H | H | H | H | H | 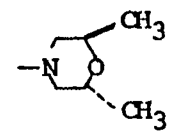 | | trans Ar/$CH_2$ | |
| 171 | $CH_2$=CH-$CH_2$- | H | H | H | H | H | H | H | 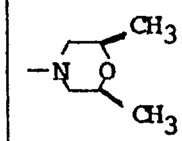 | | trans Ar/$CH_2$ | |
| 172 | $CH_2$=CH-$CH_2$- | H | H | H | H | H | H | H | 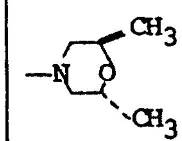 | | trans Ar/$CH_2$ | |
| 173 | CH≡C-$CH_2$- | H | H | H | H | H | H | H | 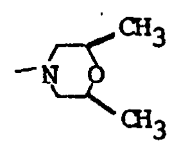 | | trans Ar/$CH_2$ | |
| 174 | CH≡C-$CH_2$- | H | H | H | H | H | H | H | 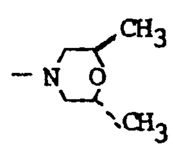 | | trans Ar/$CH_2$ | |

* protons in the piperidine ring α to the nitrogen atom.

\+ protons in the morpholine ring α to the oxygen atom.

The $^{13}C$ nmr chemical shifts for compounds of Formula (I) where $R^1$-$R^6$ equals hydrogen,

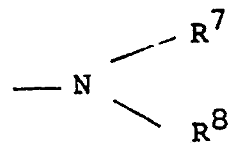

is cis 2,6-dimethylmorpholine, and Ar/$CH_2$ is trans are given in Table II. By Ar/$CH_2$ is intended, with reference to formula I, the disposition of the aryl group

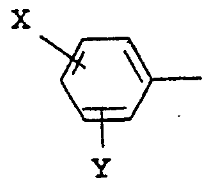

at the left hand side of the cyclopropane ring in relation to the moiety

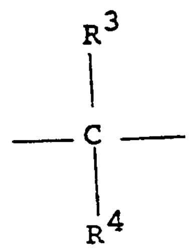

(which is -$CH_2$- since $R^3$ and $R^4$ are both H) at the right hand side of the cyclopropane ring; i.e. these are trans to each other.

TABLE II

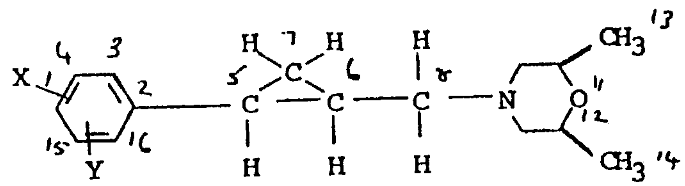

Cis $CH_3/CH_3$

Trans $Ar/CH_2$

$^{13}C$ nmr shifts (ppm from TMS)

| COMPOUND NO FROM TABLE I | X | Y | $C_1$ | $C_2$ | $C_3$ | $C_4$ | $C_5$ | $C_6$ | $C_7$ | $C_8$ |
|---|---|---|---|---|---|---|---|---|---|---|
| 4 | 4-t-$C_4H_9$ | H | 147.998 | 139.377 | 125.145 | 124.927 | 21.918 | 20.088 | 14.665 | 62.942 |
| 27 | 4-i-$C_3H_7$ | H | 146.106 | 139.998 | 126.354 | 125.610 | 22.197 | 20.181 | 14.944 | 63.097 |
| 39 | 4-t-$C_4H_9O$ | H | 153.187 | 137.446 | 125.956 | 125.933 | 21.979 | 20.378 | 14.838 | 63.069 |
| 44 | 4-$CH_3$ | H | 134.943 | 139.625 | 125.548 | 128.990 | 22.290 | 20.305 | 14.758 | 63.128 |
| 48 | 4-Br | H | 119.072 | 141.932 | 127.405 | 131.390 | 22.207 | 20.947 | 15.240 | 62.940 |
| 52 | 3-$OC_6H_5$ | H | 115.998 | 145.073 | 120.560 | 129.553 | 22.541 | 20.917 | 15.240 | 62.948 |
| 55 | H | H | 125.393 | 142.633 | 125.517 | 128.214 | 22.507 | 20.522 | 14.944 | 62.973 |
| 74 | 4-$OC_6H_5$ | H | 157.657 | 137.742 | 126.927 | 119.133 | 21.979 | 20.408 | 14.982 | 63.069 |
| 66 | 4-Cl | H | 131.048 | 141,287 | 128.308 | 128.377 | 22.040 | 20.856 | 15,164 | 62.856 |
| 78 | 4-$C_6H_5$ | H | 140.971 | 138.521 | 126.037 | 127.058 | 22.362 | 20.760 | 15.185 | 63.065 |
| 70 | 3-$OCH_3$ | H | 110.814 | - | 118.147 | 129.297 | 22.685 | 20.628 | 15.116 | 63.843 |

TABLE II CONT/D

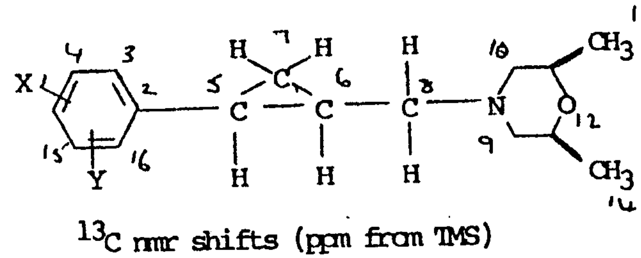

Cis $CH_3/CH_3$

Trans $Ar/CH_2$

$^{13}C$ nmr shifts (ppm from TMS)

| COMPOUND NO FROM TABLE I | X | Y | $C_9$ | $C_{10}$ | $C_{11}$ | $C_{12}$ | $C_{13}$ | $C_{14}$ | $C_{15}$ | $C_{16}$ |
|---|---|---|---|---|---|---|---|---|---|---|
| 4 | 4-t-$C_4H_9$ | H | 59.221 | 59.532 | 71.345 | 71.345 | 19.065 | 19.065 | 124.927 | 125.145 |
| 27 | 4-i-$C_3H_7$ | H | 59.346 | 59.625 | 71.593 | 71.593 | 19.220 | 19.220 | 125.610 | 126.354 |
| 39 | 4-t-$C_4H_9O$ | H | 59.441 | 59.707 | 71.599 | 71.577 | 19.224 | 19.224 | 124.119 | 125.958 |
| 44 | 4-$CH_3$ | H | 59.377 | 59.687 | 71.624 | 71.624 | 19.220 | 19.220 | 128.990 | 125.548 |
| 48 | 4-Br | H | 59.456 | 59.692 | 71.668 | 71.668 | 19.285 | 19.285 | 131.390 | 127.405 |
| 52 | 3-$OC_6H_5$ | H | 59.396 | 59.638 | 71.630 | 71.599 | 19.232 | 19.232 | 157.369 | 116.142 |
| 55 | H | H | 59.315 | 59.594 | 71.500 | 71.500 | 19.158 | 19.158 | 128.214 | 125.517 |
| 74 | 4-$OC_6H_5$ | H | 59.434 | 59.692 | 71.683 | 71.660 | 19.277 | 19.277 | 119.133 | 126.927 |
| 66 | 4-Cl | H | 59.358 | 59.593 | 71.554 | 71.554 | 19.217 | 19.217 | 128.377 | 128.308 |
| 78 | 4-$C_6H_5$ | H | 59.438 | 59.705 | 71.608 | 71.608 | 19.236 | 19.236 | 127.058 | 126.037 |
| 70 | 3-$OCH_3$ | H | 59.416 | 59.683 | 71.633 | 71.633 | 19.230 | 19.230 | 159.762 | 111.536 |

Compounds of general formula (I) :

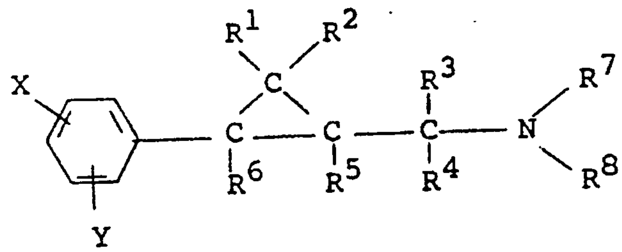

(I)

can be prepared by treatment of a compound of general formula (II) :

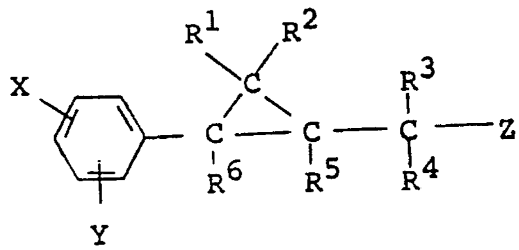

(II)

wherein $R^1$, $R^2$, $R^3$, $R^4$, $R^5$, $R^6$, X, and Y are as defined above and Z is a leaving group such as chlorine, bromine, mesylate or tosylate, with an amine of general formula (III) :

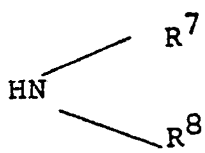

(III)

wherein $R^7$ and $R^8$ are as defined above, in the presence of a convenient solvent such as ethanol or tetrahydrofuran, or, preferably, in the absence of a solvent at a temperature of 20-100 °C. The compounds (II) can be prepared by treating an alcohol of general formula (IV) :

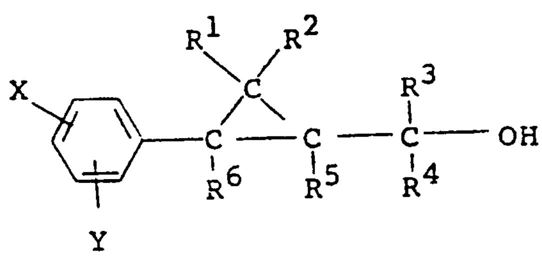

(IV)

wherein $R^1$, $R^2$, $R^3$, $R^4$, $R^5$, $R^6$, X, and Y are as defined above with the usual halogenating agents (eg. phosphorus trichloride, phosphorus pentachloride, or phosphorus oxychloride when Z = chlorine and phosphorus tribromide, phosphorus pentabromide, or phosphorus oxybromide when Z = bromine), or mesyl chloride in pyridine (or triethylamine) when Z = mesylate, or tosyl chloride in pyridine (or

triethylamine) when Z = tosylate.

The alcohols (IV), wherein $R^3$ = $R^4$ = hydrogen, can be prepared by treating an ester of general formula (V) :

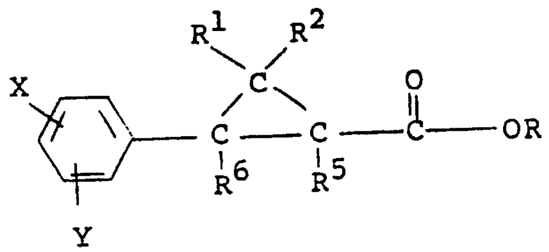

(V)

wherein $R^1$, $R^2$, $R^5$, $R^6$, X, and Y are as defined above and R is alkyl with a reducing agent (usually lithium aluminium hydride) in a convenient solvent such as diethyl ether or tetrahydrofuran.

The alcohols (IV), wherein $R^3$ = $R^4$ = alkyl, can be prepared by treating the above ester (V) with an excess of alkyl magnesium halide or an excess of alkyl lithium in a convenient solvent such as diethyl ether or tetrahydrofuran.

The esters (V) can be prepared by reacting the cinnamic esters of general formula (VI) :

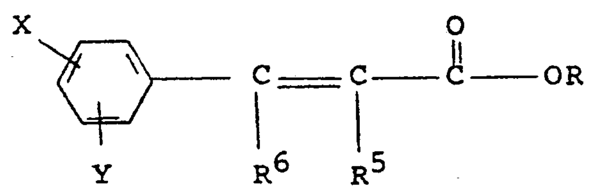

(VI)

wherein R, $R^5$ and $R^6$, X, and Y are as defined above with a phosphorane of general formula (VII) :

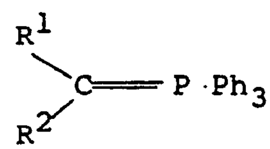

(VII)

wherein $R^1$ and $R^2$ are defined above in a convenient solvent such as tetrahydrofuran.

In an alternative process the esters of general formula (V) wherein $R^5$ = hydrogen, can be prepared by adding ethyl diazoacetate to the olefin of general formula (VIII) :

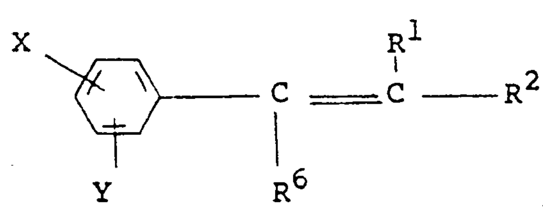

(VIII)

wherein $R^1$, $R^2$, $R^6$, X and Y are as defined above in a convenient solvent such as chloroform or dichloromethane using an appropriate catalyst such as anhydrous copper sulphate.

The compounds of general formula (I), where $R^3 = R^4$ = hydrogen, can also be prepared by treating an amide of general formula (IX) :

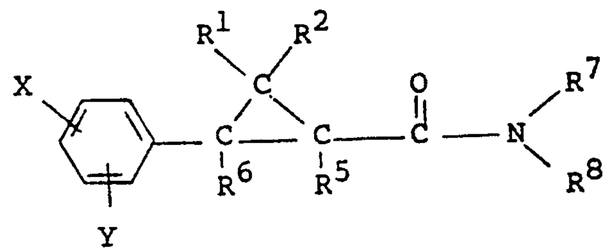

(IX)

wherein $R^1$, $R^2$, $R^5$, $R^6$, $R^7$, $R^8$, X, and Y are as defined above with a reducing agent (usually lithium aluminium hydride) in a convenient solvent such as diethyl ether or tetrahydrofuran.

The amides of general formula (IX) can be prepared by reacting an acid chloride of general formula (X) :

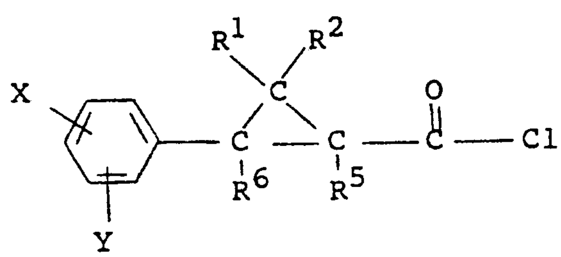

(X)

wherein $R^1$, $R^2$, $R^5$, $R^6$, X, and Y are as defined above, with an amine of general formula (III) :

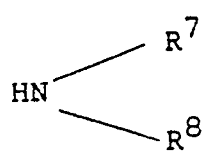

(III)

in a convenient solvent such as diethyl ether or tetrahydrofuran.

The acid chloride of general formula (V) can be prepared by reacting an acid of general formula (XI) :

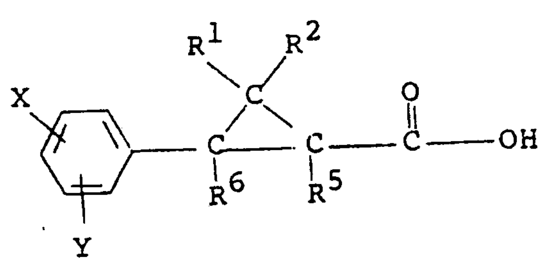

(XI)

wherein $R^1$, $R^2$, $R^5$, $R^6$, X, and Y are as defined above with the usual chlorinating agents such as thionyl chloride or oxalyl chloride in a convenient solvent such as hexane or dichloromethane.

The acid of general formula (XI) can be prepared by hydrolysing the ester (V) in the normal manner using either sodium or potassium hydroxide in methanol/water solution at room temperature.

It is normal for compounds of general formula (IV) when $R^1 = R^2$ = halogen to be prepared from the substituted allyl alcohol (XII) :

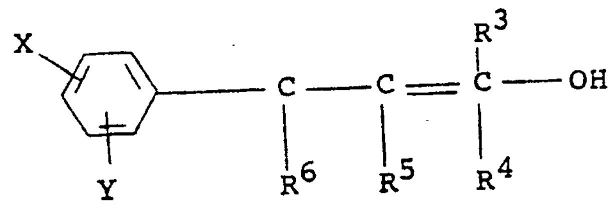

(XII)

wherein $R^3$, $R^4$, $R^5$, $R^6$, X, and Y are as defined above by the addition of a dihalocarbene in a convenient solvent such as chloroform or dichloromethane.

The compounds (VI), (VIII), and (XII) can be prepared by standard methods set out in the literature.

The salts of compounds of the general formula (I) can be prepared from the latter by known methods.

The compounds, salts and metal comlexes are active fungicides, particularly against the diseases Puccinia recondita , Puccinia striiformis and other rusts on wheat, Puccinia hordei , Puccinia striiformis and other rusts on barley, and rusts on other hosts e.g. coffee, apples, apples, vegetables and ornamental plants Erysiphe graminis (powdery mildew) on barley and wheat and other powdery mildews on various hosts such as
Sphaerotheca fuliginea on cucurbits (e.g. cucumber),
Podosphaera leucotricha on apples and Uncinula necator on vines
Helminthosporium spp., eg. Pyrenophora teres
Rhynchosporium spp. and
Pseudocercosporella herpotrichoides on cereals
Cercospora arachidicola on peanuts and other Cercospora species on for example sugar beet, bananas and soya beans Botrytis cinerea (grey mould) on tomatoes, strawberries, vines and other hosts
Venturia inaequalis (scab) on apples.

Some of the compounds have also shown a broad range of activities against fungi in vitro . They have activity against various post-harvest diseases on fruit (e.g. Penicillium digatatum and italicum on oranges and Gloeosporium musarum on bananas). Further some of the compounds are active as seed dressings against:
Fusarium spp., Septoria spp., Tilletia spp. (i.e. bunt, a seed borne disease of wheat), Ustilago spp., Helminthosporium spp. and Pseudocercosporella herpotrichoides on cereals, Rhizoctonia solani on cotton and Corticium sasakii on rice.

The compounds can move acropetally in the plant tissue. Moreover, the compounds can be volatile enough to be active in the vapour phase against fungi on the plant.

They may also be useful as industrial (as opposed to agricultural) fungicides, e.g. in the prevention of fungal attack on wood, hides, leather and especially paint films.

The compounds may be used as such for fungicidal purposes but are more conveniently formulated into compositions for such usage. The invention thus provides a fungicidal composition comprising a compound of general formula (I) as hereinbefore defined, or a salt, metal complex, ether or ester thereof; and, optionally, a carrier or diluent.

The invention also provides a method of combating fungi, which comprises applying to a plant, to seed of a plant, or to the locus of the plant or seed, a compound, or salt, metal complex, ether or ester thereof, as hereinbefore defined.

The compounds, salts, metal complexes, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a

composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethyl-ammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of

such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg. compounds having similar or complementary fungicidal activity or compounds having plant growth regulating, herbicidal or insecticidal activity.

The other fungicidal compound can be, for example, one which is capable of combating ear diseases of cereals (eg. wheat) such as Septoria , Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are carbendazim, benomyl, thiophanate-methyl, thiabendazole, fuberidazole, etridazole, dichlofluanid, cymoxanil, oxadixyl, ofurace, metalaxyl, furalaxyl, benalaxyl, fosetyl aluminium, fenarimol, iprodione, procymidione, vinclozolin, penconazole, myclobutanil, R0151297, S3308, pyrazophos, ethirimol, ditalimfos, tridemorph, triforine, nuarimol, triazbutyl, guazatine, propiconazole, prochloraz, flutriafol, chlortriafol ie. the chemical 1-(1,2,4-triazol-1-yl)-2-(2,4-dichlorophenyl)-hexan-2-ol, DPX H6573(1-((bis- 4-fluorophenyl)methylsilyl)methyl)-1H-1,2,4-triazole, triadimefon, triadimenol, diclobutrazol, fenproprimorph, fenpropidine, tridemorph, imazalil, fenfuram, carboxin, oxycarboxin, methfuroxam, dodemorph, BAS 454, blasticidin S, Kasugamycin, edifenphos, kitazin P, phthalide, probenazole, isoprothiolane, tricyclazole, pyroquilan, chlorbenzthiazone, neoasozin, polyoxin D, validamycin A, repronil, flutolanil, pencycuron, diclomenzine, phenazin oxide, nickel dimethyldithiocarbamate, techlofthalam, bitertanol, bupirimate, etaconazole, streptomycin, cypofuram, biloxazol, quinomethionate, dimethirimol, fenapanil, tolclofos-methyl, pyroxyfur, polyram, maneb, mancozeb, captafol, chlorothalonil, anilazine, thiram, captan, folpet, zineb, propineb, sulphur, dinocap, binapacryl, nitrothal-isopropyl, dodine, dithianon, fentin hydroxide, fentin acetate, tecnazene, quintozene, dichloran, copper containing compounds such as copper oxychloride, copper sulphate and Bordeaux mixture, and organomercury compounds such as 1-(2-cyano-2methoxyimino- acetyl)-3-ethyl urea.

The compounds of general formula (I) can be mixed with soil, peat or other rooting media for the protection of plants against seed-borne, soil-borne or foliar fungal diseases.

Suitable insecticides are pirimicarb, dimethoate, demeton-s-methyl, formothion, carbaryl, isoprocarb, XMC, BPMC, carbofuran, carbosulfan, diazinon, fenthion, fenitrothion, phenthoate, chlorphyrifos, isoxathion, propaphos, monocrothophas, buprofezin, ethroproxyfen and cycloprothrin.

The other plant growth regulating compound can be one which controls weeds or seedhead formations, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will be herbicides.

Examples of suitable plant growth regulating compounds, which can display synergy in admixture, or use, with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, paclobutrazol, flurprimidol, fluoridamid, mefluridide, substituted quaternary ammonium and phosphonium compounds (eg. chlormequat* chlorphonium or mepiquatchloride), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg. bromoxynil), difenzoquat, benzoylprop-ethyl 3,6-dichloropicolinic acid, paclobutrazol, fenpentezol, inabenfide, triapenthenol and tecnazene.

The following Examples illustrate the invention; the temperatures are given in degrees Centrigrade (°C).

EXAMPLE 1

This Example illustrates the preparation of 4-[2-(p-t-butylphenyl)-cyclopropylmethyl]-piperidine (Compound No 1 in Table 1). Ethyl diazoacetate (9.74g, 0.085 mol) in dichloromethane (200ml) was added dropwise to a solution of p-t-butylstyrene (13.51g, 0.085 mol) and anhydrous copper sulphate (0.1g) in dichloromethane

(50ml) at 90° C by means of a syringe pump over a period of 4 hours. The dichloromethane was distilled over during the addition. After complete addition the remaining dichloromethane was removed in vacuo and the resulting brown oil purified by column chromatography (silica eluted with petroleum ether: ethyl acetate = 4:1) to give ethyl-2-(p-t-butylphenyl)-cyclopropanecarboxylate (80%) as a yellow liquid.

Ethyl 2-(p-t-butylphenyl)-cyclopropanecarboxylate (10.23g, 0.042 mol) was added dropwise to a suspension of lithium aluminium hydride (1.9g, 0.05 mol) in sodium-dried diethyl ether at room temperature. After complete addition the solution was stirred for a further 2 hours at room temperature. Ethyl acetate (25ml) was carefully added dropwise, the mixture poured into water and extracted with diethyl ether (2 x 100ml). The ethereal extracts were washed with water, dried over anhydrous sodium sulphate, and the solvent evaporated to give 2-(p-t-butylphenyl)-cyclopropylmethanol (90%) as a colourless liquid. Methanesulphonyl chloride (5.92g, 0.052 mol) was added dropwise to a solution 2-(p-t-butylphenyl)-cyclopropylmethanol (4.79g, 0.023 mol) in pyridine (50ml) and stirred at room temperature for 10 hours. The mixture was poured into water, extracted with diethyl ether (2 x 50ml); the ethereal extracts were washed with water (4 x 75ml) and dried over anhydrous sodium sulphate. Removal of the solvent gave 2-(p-t-butylphenyl)-cyclopropylmethylchloride as a brown liquid which was used without purification in the final stage.

A solution of 2-(p-t-butylphenyl)-cyclopropylmethylchloride (0.53g, 0.0024 mol) and piperidine (1ml, 0.01 mol) in pyridine (10ml) was heated to 80° C for 10 hours. After cooling to room temperature the mixture was poured into water and extracted with diethyl ether. The ethereal extracts were washed with water, dried over anhydrous sodium sulphate, and the solvent removed to give an orange oil. Purification of this oil using column chromatography (silica gel eluted with petroleum ether: ethyl acetate = 1:1) gave the title compound as a pale yellow oil (0.3g, cis : trans $CH_2$/Ar = 1:9).

EXAMPLE 2

This Example illustrates the preparation of 4-[2-(p-t-butylphenyl)-3,3-dimethylcyclopropylmethyl]-morpholine (Compound No 8 in Table 1). n-Butyl lithium (30ml, 0.048 mol) was added dropwise to a suspension of isopropyltriphenylphosphonium idodide (20.4g, 0.047 mol) in dry tetrahydrofuran (50ml) at -5° C. After complete addition, the solution was stirred for 40 minutes at -5° C and p-t-butyl-ethylcinnamate (10g, 0.043 mol) in dry tetrahydrofuran (10ml) added dropwise at -5° C. The solution was allowed to warm upto room temperature, stirred at room temperature for 5 hours, poured into water and extracted with diethyl ether. The ethereal extracts were washed with water, dried over anhydrous sodium sulphate, and evaporated to give an orange oil. This oil was dissolved in chloroform (50ml), stirred with cuprous chloride (5g), and the chloroform evaporated. The residue was dissolved in ether (50ml), filtered, and the ether removed to give ethyl 2-(p-t-butylphenyl)-3,3-dimethylcyclopropanecarboxylate (12.2g) as a brown oil, used in the next stage without further purification. Ethyl-2(p-t-butylphenyl)-3,3-dimethylcyclopropanecarboxylate (8.7g, 0.032 mol) was added dropwise to a stirred suspension of lithium aluminium hydride (1.2g, 0.032 mol) in diethyl ether (30ml) at room temperature. After stirring at room temperature for 4 hours, ethyl acetate (15ml) was added, the mixture poured into water (50ml) and extracted with diethyl ether (2 x 50ml). The ethereal extracts were washed with water (4 x 50ml), dried over anhydrous sodium sulphate, and the solvent removed to give 2-(p-t-butylphenyl)-3,3-dimethylcyclopropanemethanol (7.5g) as a yellow oil.

Methanesulphonyl chloride (3.5ml) was added dropwise to a stirred solution of 2-(p-t-butylphenyl)-3,3-dimethylcyclopropanemethanol (2.2g, 0.04 mol) in pyridine (35ml) at 10° C. The mixture was stirred at room temperature after the addition for 10 hours, poured into 10N HCl (50ml), and extracted with diethyl ether (2 x 75ml). The combined ethereal extracts were washed with water (4 x 75ml), dried anhydrous sodium sulphate, and the solvent removed to give a colourless oil. Purified by hplc (silica eluted with hexane) to give 2-(p-t-butylphenyl)-3,3-dimethylcyclopropanemethyl chloride (3.5g).

A mixture of 2-(p-t-butylphenyl)-3,3-dimethylcyclopropanemethyl chloride (0.8g, 0.003 mol), morpholine (0.52g, 0.006 mol), and triethylamine (0.6g, 0.006 mol) were stirred at 80° C for 10 hours. The solution was cooled to room temperature, poured into water (20ml). The ethereal extracts were washed with water, and the solvent removed to give the title compound (0.91g) as a colourless oil.

EXAMPLE 3

This Example illustrates the preparation of 4-[2-(p-t-butylphenyl)-trans -cyclopropylmethyl]-2,6-cis -dimethylmorpholine (Compound No 4 in Table I). Ethyl 2-(p-t-butylphenyl)-cyclopropanecarboxylate (8.0g, 0.0325 mol) was dissolved in methanol (80ml) and a solution of potassium hydroxide (3.65g, 0.065 mol) in

water (80ml) added dropwise at room temperature. The solution was refluxed for 3 hours, cooled to room temperature and carefully neutralized with 2M HCl. Saturated sodium chloride was added; the aqueous solution extracted with ethyl acetate (4 x 100ml) and dried over anhydrous sodium sulphate. Removal of the solvent gave 2-(p-t-butylphenyl)-cyclopropanecarboxylic acid (7.8g) as a white crystalline solid m.p. 55-60 °C.

A mixture of 2-(p-t-butylphenyl)-cyclopropanecarboxylic acid (7.8g, 0.036 mol) and oxalyl chloride (5.5g, 0.043 mol) in hexane (80ml) was stirred at room temperature for 3 hours and then warmed at 60 °C for 2 hours. The hexane and excess oxalyl chloride were removed in vacuo to give 2-(p-t-butylphenyl)-cyclopropanecarbonyl chloride as a pale yellow liquid which was used in the next stage without further purification.

2,6-Dimethylmorpholine (9.2g, 0.008 mol) was added dropwise to a solution of 2-(p-t-butylphenyl)-cyclopropanecarbonyl chloride (4.96g, 0.02 mol) in sodium dried ether (40ml) at 10 °C and after complete addition the solution was stirred at 20 °C for 3 hours. The reaction was poured into water and extracted with diethyl ether (2 x 100ml). The ethereal extracts were washed with water, dried over anhydrous sodium sulphate, and the solvent removed to give [2-(p-t-butylphenyl)-cyclopropane] carbonyl 2,6-dimethylmorpholine (5.6g) as an orange oil.

A solution of [2-(p-t-butylphenyl)-cyclopropane] carbonyl 2,6-dimethylmorpholine (1.35g, 0.0043 mol) in sodium-dried diethyl ether (10ml) was added dropwise to a suspension of lithium aluminium hydride (0.2g, 0.0053 mol) in sodium-dried diethyl ether (20ml) and the mixture stirred at 20 °C for 3 hours. The reaction mixture was poured into water and extracted with diethyl ether. The ethereal extracts were washed with water, dried over anhydrous sodium sulphate, and the solvent removed to give a colourless oil. This oil was purified by column chromatography (silica gel eluted with ethyl acetate/petroleum ether 1:1) to give the title compound (0.48g) as a colourless oil.

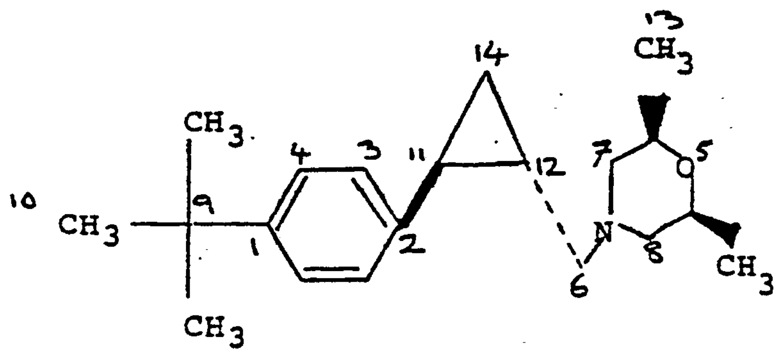

$^{13}C$ nmr (Shifts in ppm from TMS)

| | | | |
|---|---|---|---|
| C - 1 | 147.998 | C - 8 | 59.221 |
| C - 2 | 139.377 | C - 9 | 34.075 |
| C - 3 | 125.145 | C - 10 | 31.222 |
| C - 4 | 124.927 | C - 11 | 21.918 |
| C - 5 | 71.345 | C - 12 | 20.088 |
| C - 6 | 62.942 | C - 13 | 19.065 |
| C - 7 | 59.532 | C - 14 | 14.665 |

EXAMPLE 4

This Example illustrates the preparation of 4-[2-(p-trimethylsilylphenyl)-trans -cyclopropylmethyl]-2,6-cis -dimethylmorpholine (Compound No. 133 in Table I). n-Butyl lithium (0.12 mol, 73.6 ml of 1.6M solution) was added dropwise at -60 ° - -70 °C to a solution of p-bromostyrene (21.6g, 0.12 mol) in dry tetrahydrofuran (160 ml) under nitrogen. After complete addition the reaction mixture was stirred at -60 °C

for 1½ hours, and trimethylsilyl chloride (12.8g, 0.12 mol) added dropwise at -50° C and stirred for a further ½ hour at -50° C after the addition. The mixture was stirred at room temperature for 1 hour, poured carefully into water, and extracted with diethyl ether. The ethereal extracts were washed with water (x3), dried over anhydrous sodium sulphate, and the solvent removed to give an orange oil. Purification by column chromatography [silica gel eluted with petroleum ether (bp. 40-60° C)] gave p-trimethylsilylstyrene (18.90g, 91.2%) as a colourless liquid.

Ethyl diazoacetate (17.6g, 0.15 mol) in dry dichloromethane (250 ml) was added dropwise to a solution of p-trimethylsilylstyrene (18.13g, 0.103 mol) and anhydrous copper sulphate (0.4g) in dry dichloromethane (50 ml) at 90° C by means of a syringe pump over a period of 5½ hours. The dichloromethane was distilled over during the addition. After complete addition the remaining dichloromethane was removed in vacuo and the resulting brown oil purified by column chromatography (silica gel eluted with petroleum ether:ethyl acetate = 9:1) to give ethyl-2-(p-trimethylsilylphenyl)-cyclopropanecarboxylate (13.8g, 51%).

Ethyl-2-(p-trimethylsilylphenyl)-cyclopropanecarboxylate (13.7g, 0.052 mol) was dissolved in methanol (125 ml) and a solution of potassium hydroxide (5.86g, 0.105 mol) in water (125 ml) added dropwise at room temperature. The solution was refluxed for 5 hours, cooled to room temperature and carefully neutralised with 2M HCl. Saturated sodium chloride was added; the aqueous solution extracted with ethyl acetate (4 x 150 ml) and dried over anhydrous sodium sulphate. Removal of the solvent gave 2-(p-trimethylsilyl- phenyl)-cyclopropanecarboxylic acid (11.6g, 95%) as a white crystalline solid.

A mixture of 2-(p-trimethylsilylphenyl)-cyclopropanecarboxylic acid (11.6g, 0.05 mol) and oxalyl chloride (18.9g, 0.15 mol) in hexane (90 ml) was stirred at room temperature for 1 hour and then warmed at 60° C for 5 hours. The hexane and excess oxalyl chloride were removed in vacuo to give 2-(p-trimethylsilyl-phenyl)-cyclopropanecarbonyl chloride (12.2g, 97.5%) as a pale yellow liquid which was used in the next stage without further purification.

2,6-Dimethylmorpholine (22.2g, 0.19 mol) was added dropwise to a solution of 2-(p-trimethylsilylphenyl)-cyclopropanecarbonyl chloride (12.2g, 0.048 mol) in sodium dried ether (100 ml) at 10° C and after complete addition the solution was stirred at 20° C for 3 hours. The reaction mixture was poured into water and extracted with diethyl ether (2 x 150 ml). The ethereal extracts were washed with water, dried over anhydrous sodium sulphate, and the solvent removed to give 2-(p-trimethylsilylphenyl)-cyclop ropanecarbonyl-2,6-dimethylmorpholine (16.1g).

A solution of 2-(p-trimethylsilylphenyl)-cyclopropanecarbonyl-2,6-dimethylmorpholine(3.94g, 0.012 mol) in dry sodium-dried diethyl ether (25 ml) was added dropwise to a suspension of lithium aluminium hydride (0.68g, 0.18 mol) in sodium-dried diethyl ether (15 ml) and the mixture stirred at 20° C for 3 hours. The reaction mixture was poured into water and extracted with diethyl ether. The ethereal extracts were washed with water, dried and anhydrous sodium sulphate, and the solvent removed to give a colourless oil. This oil was purified by column chromatography (silica gel eluted with ethyl acetate/ petroleum ether 1:1) to give the title compound (2.0g). Two further isomers (compound Nos. 132 and 134 in Table I) were also isolated.

EXAMPLE 5

This Example illustrates the preparation of 4-[2-(p-t -butylphenyl)-2-methyl-trans -cyclopropylmethyl]-2,6-cis -dimethylmorpholine (Compound No. 15 in Table I). Sodium hydride (5.45g-50% suspension in oil, 0.11 mol) was washed with petroleum ether, suspended in dry DMSO (90 ml) and heated with stirring at 60°-70° C for 2½ hours. The reaction mixture was then cooled to 15° C and methyl triphenylphosphonium bromide (40.1g, 0.11 mol) was added portionwise with rapid stirring over a ½ hour period. The reaction mixture was stirred at room temperature for a further 1 hour after the addition and 4-t -butylacetophenone (20.0g, 0.11 mol) was added dropwise over a period of 1 hour. The resultant reaction mixture was stirred at room temperature for 12 hours, poured into water (250 ml) and extracted with ether (4 x 150 ml). The ethereal extracts were washed with water (4 x 100 ml) and dried over anhydrous sodium sulphate. Removal of the solvent gave an orange oil which was purified by column chromatography (silica gel eluted with petroleum ether) to give α-methyl-4-t -butylstyrene (17.83g, 90%) as a colourless liquid.

Ethyl diazoacetate (13.9g, 0.12 mol) in dry dichloromethane (200 ml) was added dropwise to a solution of α-methyl-4-t -butylstyrene (17.65g, 0.10 mol) and anhydrous copper sulphate (0.3g) in dry dichloromethane (50 ml) at 90° C by means of a syringe pump over a period of 5½ hours. The dichloromethane was distilled over during the addition. After complete addition the remaining dichloromethane was removed in vacuo and the resulting brown oil purified by column chromatography (silica gel eluted with petroleum ether:ethyl acetate = 9.1) to give ethyl-2-(p-t -butylphenyl)-2-methylcyclopropanecarboxylate (19.3g, 73.6%) as a colourless oil.

Ethyl-2-(p-t -butylphenyl)-2-methylcyclopropanecarboxylate (18.9g, 0.073 mol) was dissolved in methanol (165 ml) and a solution of potassium hydroxide (8.13g, 0.145 mol) in water (165 ml) added dropwise at room temperature. The solution was refluxed for 4 hours, cooled to room temperature and carefully neutralised with 2 M HCl. Saturated sodium chloride was added; the aqueous solution extracted with ethyl acetate (4 x 150 ml) and dried over anhydrous sodium sulphate. Removal of the solvent gave 2-(p-t-butylphenyl)-2-methylcyclopropanecarboxylic acid (16.9g, 100%) as an off-white solid.

A mixture of 2-(p-t -butylphenyl)-2-methylcyclopropanecarboxylic acid (16.2g, 0.07 mol) and oxalyl chloride (12.3g, 0.1 mol) in hexane (130 ml) was stirred at room temperature for 1 hour and then warmed at 60°C for 5 hours. The hexane and excess oxalyl chloride were removed in vacuo to give 2-(p-t -butylphenyl)-2-methylcyclopropanecarboxylic chloride (17.3g, 100%) as a pale yellow liquid which was used in the next stage without further purification.

2,6-Dimethylmorpholine (15.6g, 0.14 mol) was added dropwise to a solution of 2-(p-t -butylphenyl)-2-methylcyclopropane carbonyl chloride (8.6g, 0.035 mol) in sodium dried ether (65 ml) at 10°C and after complete addition the solution was stirred at 20°C for 3½ hours. The reaction mixture was poured into water and extracted with diethyl ether (2 x 150 ml). The ethereal extracts were washed with water, dried over anhydrous sodium sulphate, and the solvent removed to give 2-(p-t -butylphenyl)-2-methylcyclopropanecarbonyl-2,6-dimethylmorpholine (12.4g, 100%).

A solution of 2-(p-t -butylphenyl)-2-methylcyclopropanecarbonyl-2,6-dimethylmorpholine (11.8g, 0.036 mol) in sodium dried ether (60 ml) was added dropwise to a suspension of lithium aluminium hydride (1.6g, 0.042 mol) in sodium-dried ether (40 ml) and the mixture stirred at room temperature for 3 hours. The reaction mixture was poured carefully into water and extracted with diethyl ether. The ethereal extracts were washed with water, dried over anhydrous sodium sulphte, and the solvent removed to give a colourless oil. This oil was purified by column chromatography (silica gel eluted with ethyl acetate/ petroleum ether 1:1) to give the title compound (1.5g). Two further isomers (compound Nos. 16 and 17 in Table I) were also isolated.

EXAMPLE 6

This Example illustrates the preparation of 4-[2-(p-t -propylphenyl)-3-methyl-trans -cyclopropylmethyl]-2,6-cis -dimethylmorpholine (Compound No. 94 in Table I). Sodium hydride (7.2g - 50% suspension in oil, 0.15 mol) was washed with petroleum ether, suspended in dry DMSO (120 ml) and heated with stirring at 60°-70° for 2 hours. The reaction mixture was then cooled to 15°C and ethyl triphenylphosphonium bromide (57.4g, 0.15 mol) was added portionwise with rapid stirring over a ½ hour period. The reaction mixture was stirred at room temperature for a further 1 hour after the addition and 4-i -propylbenzaldehyde (20g, 0.13 mol) was added dropwise over a period of 1 hour. The resultant reaction mixture was stirred at room temperature for 12 hours, poured into water (300 ml) and extracted with ether (4 x 150 ml). The ethereal extracts were washed with water (4 x 100 ml) and dried over anhydrous sodium sulphate. Removal of the solvent gave a yellow oil which was purified by column chromatography (silica gel eluted with petroleum ether: ethyl acetate - 9:1) to give β-methyl-i -propylstyrene (19.0g, 90%).

Ethyl diazoacetate (13.0g, 0.11 mol) in dry dichloromethane (150 ml) was added dropwise to a solution of β-methyl-i -propylstyrene (18.0g, 0.11 mol) and anhydrous copper sulphate (0.3g) in dry dichloromethane (50 ml) at 90°C by means of a syringe pump over a period of 5 hours. The dichloromethane was distilled over during the addition. After complete addition the remaining dichloromethane was removed in vacuo and the resulting brown oil purified by column chromatography (silica gel eluted with petroleum ether:ethyl acetate 1:1) to give ethyl-2-(p-i -propylphenyl)-3-methylcyclopropanecarboxylate (26.0g, 96%).

Ethyl-2-(p-i -propylphenyl)-3-methylcyclopropanecarboxylate (24.8g, 0.1 mol) was dissolved in methanol (130 ml) and a solution of potassium hydroxide (13.g, 0.23 mol) in water (130 ml) added dropwise at room temperature. The solution was refluxed for 3 hours, cooled to room temperature and carefully neutralised with 2 M HCl. Saturated sodium chloride was added, the aqueous solution extracted with ethyl acetate (4 x 150 ml) and dried over anhydrous sodium sulphate. Removal of the solvent gave 2-(p-i -propylphenyl)-3-methylcyclopropanecarboxylic acid (17.0g, 78%).

A mixture of 2-(p-i -propylphenyl)-3-methylcyclopropanecarboxylicacid (15.7g, 0.07 mol) and oxalyl chloride (10g, 0.08 mol) in hexane (120 ml) was stirred at room temperature for 2 hours and then warmed at 60°C for 3 hours. The hexane and excess oxalyl chloride were removed in vacuo to give 2-(p-i -propylphenyl)-3-methyl-cyclopropanecarbonyl chloride (17.0g, 100%) as a pale yellow liquid which was used in the next stage without further purification.

2,6-Dimethylmorpholine (5.0g, 0.043 mol) was added dropwise to a solution of 2-(p-i -propylphenyl)-3-methylcyclopropanecarbonyl chloride (5.0g, 0.021 mol) in sodium dried ether (40 ml) at 10° C and after complete addition the solution was stirred at 20° C for 3 hours. The reaction mixture was poured into water and extracted with diethyl ether (2 x 100 ml). The ethereal extracts were washed with water, dried over anhydrous sodium sulphate, and the solvent removed to give 2-(p-i -propylphenyl)-3-methylcyclopropanecarbonyl-2,6-dimethylmorpholine (6.3g, 95%) as a yellow oil.

A solution of 2-(p-t -propylphenyl-3-methylcyclopropanecarbonyl-2,6-dimethylmorpholine (5.8g, 0.018 mol) in sodium dried ether (30 ml) was added dropwise to a suspension of lithium aluminium hydride (0.8g, 0.02 mol in sodium dried ether (20 ml) and the mixture stirred at room temperature for 3 hours. The reaction mixture was poured carefully into water and extracted with diethyl ether. The ethereal extracts were washed with water, dried over anhydrous sodium sulphate, and the solvent removed to give a colourless oil. This oil was purified by column chromatography (silica gel eluted with ethyl acetate/ petroleum ether 2:3) to give the title compound (1.1g). Three further isomers (Compound Nos. 92, 93 and 95 in Table I) were also isolated.

EXAMPLE 7

An emulsifiable concentrate was made up by mixing the ingredients, and stirring the mixture until all the constituents were dissolved.

| | |
|---|---|
| Compound of Example 1 | 10% |
| Ethylene dichloride | 40% |
| Calcium dodecylbenzenesulphate | 5% |
| "Lubrol" L | 10% |
| "Aromasol" H | 35% |

EXAMPLE 8

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound of Example 2 | 50% |
| "Dispersol" T | 25% |
| "Lubrol" APN5 | 1.5% |
| Sodium acetate | 23.5% |

EXAMPLE 9

The ingredients were all ground together to produce a powder formulation readily dispersible in liquids.

| | |
|---|---|
| Compound of Example 1 | 45% |
| "Dispersol" T | 5% |
| "Lissapol" NX | 0.5% |

| | |
|---|---|
| "Cellofas" B600 | 2% |
| Sodium acetate | 47.5% |

EXAMPLE 10

The active ingredient was dissolved in a solvent and the resultant liquid was sprayed on to the granules of China clay. The solvent was then allowed to evaporate to produce a granular composition.

| | |
|---|---|
| Compound of Example 2 | 5% |
| China clay granules | 95% |

EXAMPLE 11

A composition suitable for use as a seed dressing was prepared by mixing the three ingredients.

| | |
|---|---|
| Compound of Example 1 | 50% |
| Mineral oil | 2% |
| China clay | 48% |

EXAMPLE 12

A dusting powder was prepared by mixing the active ingredient with talc.

| | |
|---|---|
| Compound of Example 2 | 5% |
| Talc | 95% |

EXAMPLE 13

A Col formulation was prepared by ball-milling the constituents set out below and then forming an aqueous suspension of the ground mixture with water.

| | |
|---|---|
| Compound of Example 1 | 40% |
| "Dispersol" T | 10% |
| "Lubrol" APN5 | 1% |
| Water | |

EXAMPLE 14

A dispersible powder formulation was made by mixing together the ingredients set out below and then

grinding the mixture until all were thoroughly mixed.

| | |
|---|---|
| Compound of Example 2 | 25% |
| "Aerosol" OT/B | 2% |
| "Dispersol" A.C. | 5% |
| China clay | 28% |
| Silica | 40% |

EXAMPLE 15

This Example illustrates the preparation of a dispersible powder formulation. The ingredients were mixed and the mixture then ground in a comminution mill.

| | |
|---|---|
| Compound of Example 1 | 25% |
| "Perminal" BX | 1% |
| "Dispersol" T | 5% |
| Polyvinylpyrrolidone | 10% |
| Silica | 25% |
| China clay | 34% |

EXAMPLE 16

The ingredients set out below were formulated into a dispersible powder by mixing then grinding the ingredients.

| | |
|---|---|
| Compound of Example 2 | 25% |
| "Aerosol" OT/B | 2% |
| "Dispersol" A | 5% |
| China clay | 68% |

In Examples 7 to 16 the proportions of the ingredients given are by by weight.

The compounds set out in Table 1 and numbered 3 to 33 can be similarly formulated as specifically described in Examples 7 to 16.

There now follows an explanation of the compositions or substances represented by the Trade Marks used in Example Numbers 7, 8, 9, 13, 14, 15 and 16 above.

| | |
|---|---|
| "LUBROL L": | a condensate of nonyl phenol 1 mole) with ethylene oxide (13 moles) |
| "ARMOMASOL H": | a solvent mixture of alkylbenzenes |
| "DISPERSOL" T & AC: | a mixture of sodium sulphate and a condensate of formaldehyde with sodium naphthalene sulphonate |
| "LUBROL" APN5: | a condensate of nonyl phenol (1 mole) with naphthalene oxide (5.5 moles) |
| "CELLOFAS" B600: | A sodium carboxymethyl cellulose thickener |
| "LISSAPOL" NX: | a condensate of nonyl phenol (1 mole) with ethylene oxide (8 moles) |
| "AEROSOL" OT/B: | dioctyl sodium sulphosuccinate |
| "PERMINAL" BX : | a sodium alkyl naphthalene sulphonate |

The words above in capital letters and enclosed within inverted commas are all Trade Marks.

EXAMPLE 17

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous Dispersol T or as a solution in acetone or acetone/ethanol which was diluted to the required concentration immediately before use. For the foliage diseases, the formulations (100 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with thedisease. An exception was the test on Erysiphe graminis in which the plants were inoculated 24 hours before treatment. Foliar pathogens were applied by spray as spore suspensions onto the leaves of test plants. After inoculation, the plants were put into an appropriate envioronment to allow infection to proceed and then incubated until the disease was ready for assessment. The period between inocultion and assessment varied from four to fourteen days according to the disease and environment.

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table III.

TABLE III

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 1 | 4 | 4 | 0 | 1 |
| 2 | 4 | 4 | 0 | 0 |
| 3 | 2 | 4 | 0 | 0 |
| 4 | 3 | 4 | 3 | 0 |
| 5 | 3 | 4 | 0 | 0 |
| 6 | 0 | 4 | 4 | 4 |
| 7 | 3 | 4 | 0 | 0 |
| 8 | 0 | 4 | 3 | 3 |
| 9 | 0 | 4 | 0 | 1 |
| 10 | 0 | 4 | 4 | 4 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 11 | 4 | 4 | 0 | 4 |
| 12 | 4 | 4 | 4 | 4 |
| 13 | 3 | 4 | 2 | - |
| 15 | 3 | 4 | 3 | - |
| 16 | 3 | 4 | - | - |
| 17 | 0 | 4 | - | - |
| 25 | 0 | 3 | 0 | 0 |
| 26 | 2 | 2 | 0 | 0 |
| 27 | 3 | 4 | 4 | 4 |
| 28 | 0 | 4 | 0 | 0 |
| 29 | 0 | 4 | 3 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 30 | 3 | 4 | 0 | 0 |
| 31 | 0 | 4 | 4 | 0 |
| 32 | 0 | 4 | 4 | 3 |
| 34 | 4 | 4 | 3 | - |
| 35 | 1 | 4 | - | - |
| 36 | 0 | 4 | 3 | - |
| 38 | 0 | 3 | 0 | 0 |
| 39 | 3 | 4 | - | - |
| 40 | 3 | 4 | - | - |
| 42 | 2 | 0 | - | - |
| 43 | 0 | 3 | - | - |
| 44 | 0 | 4 | - | - |
| 45 | 3 | 4 | 0 | 0 |
| 47 | 0 | 2 | 0 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 48 | 3 | 4 | 0 | 0 |
| 49 | 0 | 4 | 2 | 0 |
| 50 | 0 | 2 | 0 | 0 |
| 51 | 0 | 3 | 4 | 2 |
| 52 | 2 | 4 | 4 | 4 |
| 53 | 2 | 4 | 3 | 4 |
| 54 | 1 | 0 | 0 | 0 |
| 55 | 1 | 4 | 0 | 0 |
| 56 | 1 | 3 | 0 | 0 |
| 57 | 2 | 4 | 0 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 58 | 2 | 4 | - | - |
| 59 | 2 | 4 | - | - |
| 60 | 0 | 1 | 0 | - |
| 62 | 0 | 3 | 0 | 0 |
| 63 | 0 | 4 | 0 | 0 |
| 65 | 0 | 2 | 0 | 0 |
| 66 | 3 | 4 | 0 | 2 |
| 67 | 0 | 4 | 0 | 1 |
| 69 | 0 | 3 | 0 | 0 |
| 70 | 3 | 4 | 0 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 71 | 0 | 4 | 0 | 0 |
| 72 | 0 | 1 | 0 | 0 |
| 74 | 0 | 3 | 0 | 1 |
| 75 | 0 | 4 | 0 | 1 |
| 76 | 0 | 1 | 0 | 1 |
| 77 | 0 | 4 | 0 | 0 |
| 78 | 3 | 3 | 0 | 0 |
| 79 | 3 | 4 | 0 | 3 |
| 80 | 0 | 3 | 0 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 81 | 0 | 3 | 0 | 1 |
| 82 | 0 | 4 | 0 | 0 |
| 83 | 0 | 4 | 0 | 0 |
| 84 | 0 | 4 | 0 | 0 |
| 85 | 0 | 0 | 0 | 2 |
| 86 | 0 | 3 | 0 | 1 |
| 87 | 0 | 4 | 0 | 3 |
| 88 | 0 | 4 | 0 | 1 |
| 89 | 0 | 2 | 0 | 4 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 90 | 0 | 3 | 0 | 0 |
| 91 | 0 | 4 | 4 | 0 |
| 92 | 1 | 4 | 3 | 3 |
| 93 | 3 | 4 | 3 | 3 |
| 94 | 4 | 4 | 4 | 4 |
| 95 | 2 | 4 | 3 | 1 |
| 97 | 3 | 2 | 0 | 2 |
| 98 | 3 | 4 | 0 | 3 |
| 99 | 1 | 4 | 0 | 1 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 100 | 3 | 4 | 0 | 2 |
| 101 | 4 | 4 | 0 | 3 |
| 102 | 4 | 4 | 0 | 3 |
| 103 | 3 | 4 | 0 | 0 |
| 104 | 3 | 4 | 4 | 2 |
| 105* | 0 | 0 | 0 | 1 |
| 109 | 0 | 0 | 1 | 0 |
| 110 | 4 | 4 | 3 | 3 |

* 25 ppm foliar spray

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 111 | 2 | 4 | - | 1 |
| 113 | 0 | 3 | 4 | 3 |
| 114 | 0 | 3 | 3 | 1 |
| 116 | 4 | 4 | 4 | 4 |
| 117 | 0 | 4 | 3 | 2 |
| 118 | 4 | 4 | 4 | 4 |
| 119 | 3 | 4 | 4 | 4 |
| 120 | 0 | 4 | 0 | 0 |

TABLE III CONT/D

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 129 | 2 | 4 | 4 | 3 |
| 130 | 2 | 4 | 1 | 4 |
| 131 | 1 | 4 | 4 | 4 |
| 132 | 3 | 4 | 4 | 0 |
| 133 | 4 | 4 | 4 | 3 |
| 134 | 1 | 4 | 4 | 4 |

## Claims

1. A method of combating plant fungi which comprises applying to a plant, to the seed of a plant, or to th locus of the plant, or to the locus of the plant or seed a compound having the general formula (I):

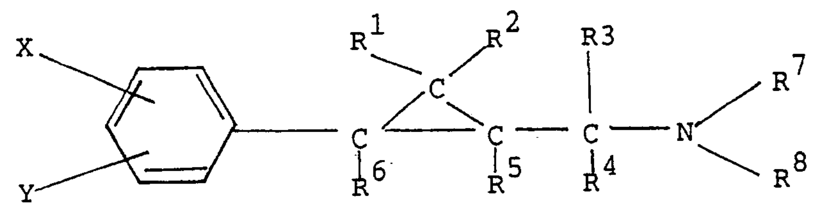 (I)

or a stereoisomer or acid addition salt thereof, wherein $R^1$, $R^2$, $R^3$, $R^4$, $R^5$ and $R^6$, which may be the same or different, represent a hydrogen atom, an alkyl group containing from 1 to 4 carbon atoms, or a halogen atom; $R^7$ and $R^8$, which may be the same or different, represent a hydrogen atom or an alkyl group containing from 1-4 carbon atoms or together form a ring which may contain oxygen, nitrogen or sulphur as an additional heteroatom, and which ring may be optionally substituted with one or more $C_{1-4}$ alkyl groups, or a phenyl group or an hydroxyalkyl group; X and Y, which may be the same of different, represent a hydrogen or a halogen atom, or an alkyl group containing from 1 to 6 carbon atoms, a cycloalkyl group containing from 3 to 6 carbon atoms, an alkenyl or alkynyl group each containing from 2 to 6. carbon atoms, a phenyl group, a benzyl group, an alkoxy group containing from 1 to 6 carbon atoms, a phenoxy group, a benzyloxy group, or a

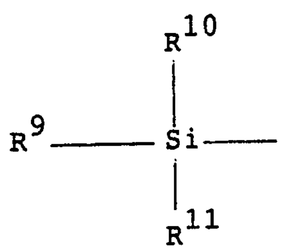

group wherein $R^9$, $R^{10}$ and $R^{11}$, which may be the same or different, represent an alkyl group containing up to 4 carbon atoms, an alkenyl or alkynyl group each containing from 2 to 4 carbon atoms, a cycloalkyl group containing from 3 to 6 carbon atoms, or a phenyl group.

**2.** Compounds of general formula (I) :

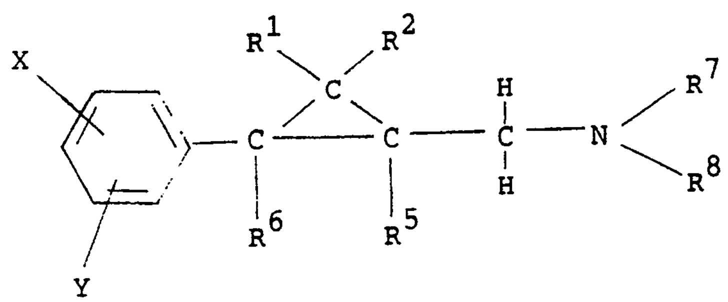

(I)

and stereoisomers and acid addition salts thereof, wherein $R^1$ and $R^2$ are hydrogen, halogen or $C1\text{-}_4$ alkyl; R5 and R6 are hydrogen or $C_{1-4}$ alkyl; $R^7$ and $R^8$ together with the adjacent N-atom, represent a piperidine, morpholine, thiomorpholine or piperazine ring which may be substituted with $C_{1-4}$ alkyl, phenyl, or hydroxymethyl; and X and Y are $C_{1-4}$ alkyl, phenyl, phenoxy, benzyl, benzyloxy or a

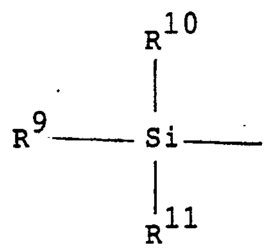

group wherein $R^9$, $R^{10}$ and $R^{11}$ are $C_{1-4}$ alkyl.

**3.** Compounds as claimed claim 3 and of general formula (I) :

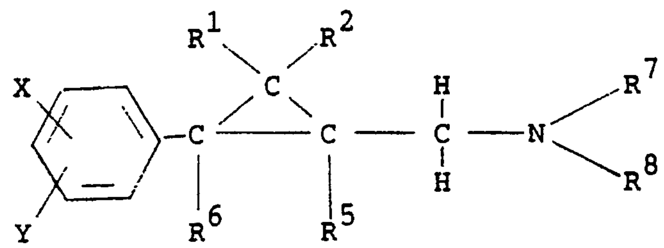

(I)

and stereoisomers and acid addition salts thereof, wherein $R^1$ and $R^2$ are hydrogen, methyl, or chlorine; $R^5$ and $R^6$ are hydrogen or methyl; $R^6$ and $R^7$ are piperidine, 3,5-dimethyl piperidine, morpholine, 2,6-dimethylmorpholine, diethylamino, 4-phenyl piperidine, 3-hydroxymethyl piperidine, thiomorpholine, piperazine, or 4-phenyl-piperazine; and X and Y are substituents at the 3- or 4-positions of the phenyl ring and one of which is hydrogen or $C_{1-4}$ alkyl whilst the other is hydrogen,$C_{1-4}$ alkyl, phenyl, phenoxy, benzyl, benzyloxy, or $C_{1-4}$ trialkylsilyl, provided that X and Y are not both hydrogen.

**4.** The compounds

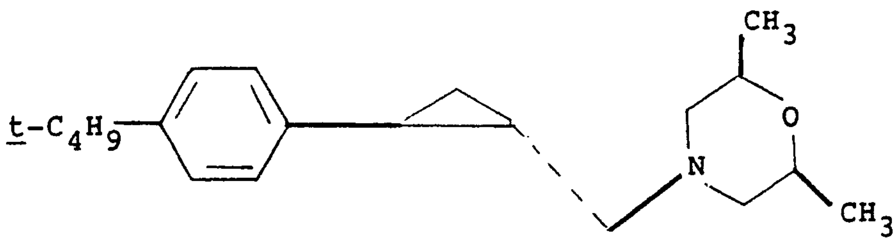

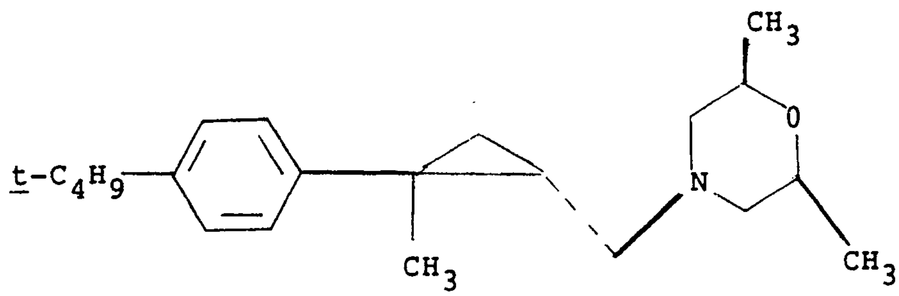

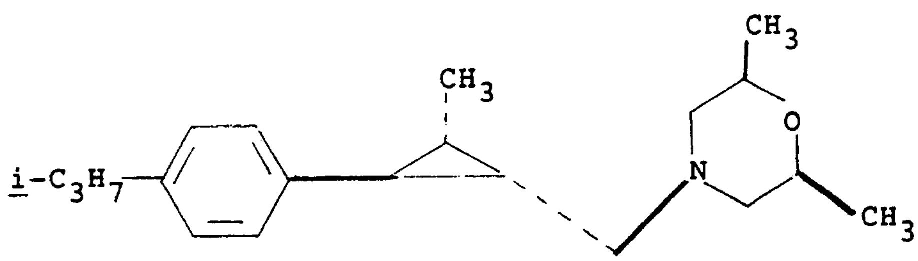

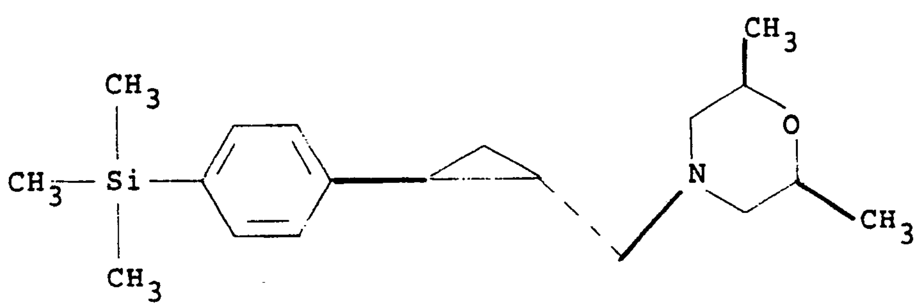

5. A process for preparing the compounds defined in claims 2 to 4 which comprises the treatment of a compound of general formula (II) :

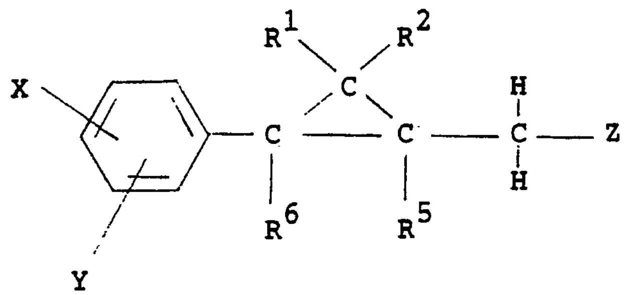

(II)

wherein $R^1$, $R^2$, $R^5$, $R^6$, X and Y are as defined and Z is a leaving group such as chlorine, bromine, mesylate or tosylate, with an amine of general formula (III) :

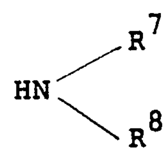

(III)

Wherein $R^7$ and $R^8$ are as defined above, in the presence of a convenient solvent such as ethanol or tetrahydrofuran, or, preferably, in the absence of a solvent at a temperature of 20 to 100 °C.

6. A process for preparing the compounds of general formula (I) as claimed in claims 2 to 4 which comprises treating an amide of general formula (IX) :

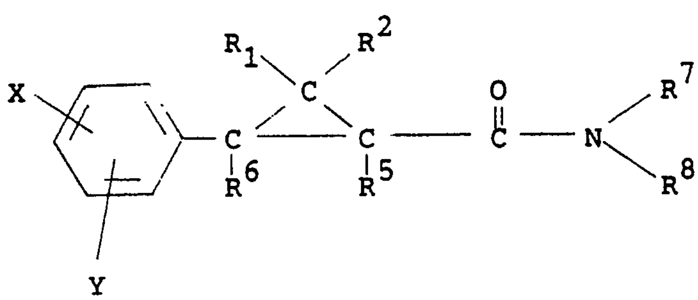

(IX)

wherein $R^1$, $R^2$, $R^5$, $R^6$, $R^7$, $R^8$, X and Y are as defined with a reducing agent (usually lithium aluminium hydride) in a convenient solvent such as diethyl ether or tetrahydrofuran.

7. A process as claimed ibn claim 6 which comprises preparing amides of general formula (IX) by reacting an acid chloride of general formula (X) :

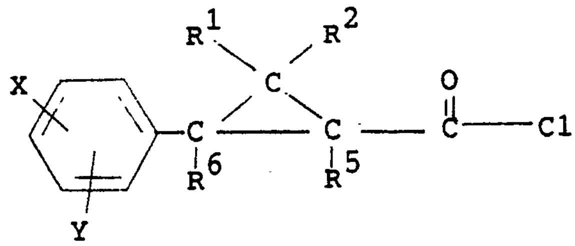

(X)

wherein $R^1$, $R^2$, $R^5$, $R^6$ X and Y are as defined with an amine of general formula (III) :

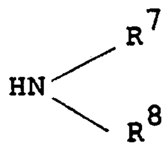

(III)

in a convenient solvent such as diethyl ether or tetrahydrofuran, the acid chloride of general formula (X) being prepared by reacting an acid of general formula (XI) :

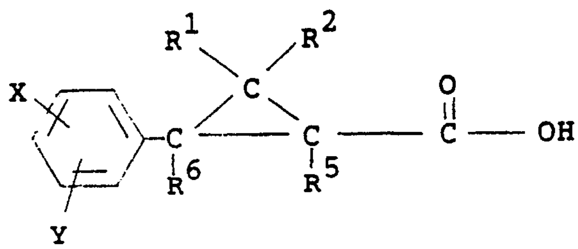

(XI)

wherein $R^1$, $R^2$, $R^5$, $R^6$, X and Y are as defined above with the usual chlorinating agents such as thionyl chloride or oxalyl chloride in a convenient solvent such as hexane or dichloromethane, the acid of general formula XI being itself prepared by hydrolysing the ester (V) in the normal manner using either sodium or potassium hydroxide in methanol/water solution at room temperature.

8. A fungicidal composition comprising a compound of general formula (I) as claimed in any of claims 2 to 4 or acid addition salt or stereoisomer theeof; and, optionally, a carrier of diluent.

9. A method of combating fungi, which comprises applying to a plant, to seed of a plant, or to the locus of a plant or seed, a compound, or an acid addition salt or stereoisomer thereof, as claimed in any of claims 2 to 4 or a composition as claimed in claim 8.

CLAIMS FOR THE FOLLOWING CONTRACTING STATE : AT

1. A process for preparing the compounds of general formula (I) :

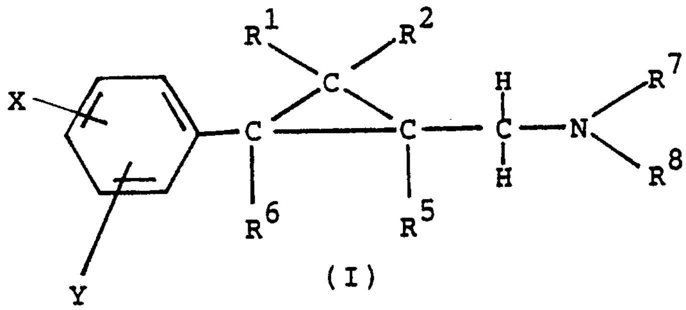

(I)

and stereoisomers and acid addition salts thereof, wherein $R^1$ and $R^2$ are hydrogen, halogen or $C_{1-4}$ alkyl; $R^5$ and $R^6$ are hydrogen or $C_{1-4}$ alkyl; $R^7$ and $R^8$ together with the adjacent N-atom, represent a piperidine, morpholine, thiomorpholine or piperazine ring which may be substituted with $C_{1-4}$ alkyl, phenyl, or hydroxymethyl; and X and Y are $C_{1-4}$ alkyl, phenyl, phenoxy, benzyl, benzyloxy or a

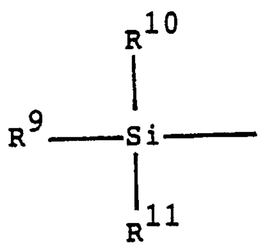

group wherein $R^9$, $R^{10}$ and $R^{11}$ are $C_{1-4}$ alkyl which comprises;

(a) the treatment of a compound of general formula (II):

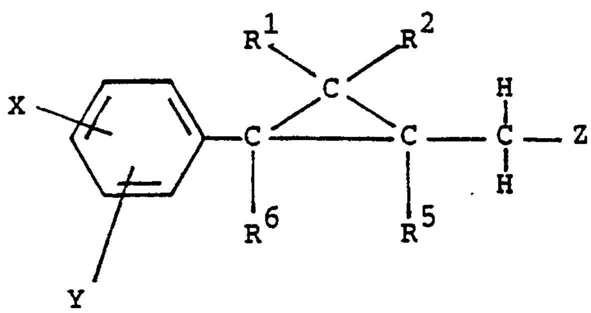

(II).

wherein $R^1$, $R^2$, $R^5$, $R^6$, X and Y are as defined above and Z is a leaving group such as chlorine, bromine, mesylate or tosylate, with an amine of general formula (III):

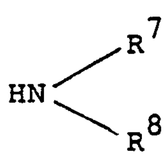

(III)

wherein $R^7$ and $R^8$ are as defined above, in the presence of a convenient solvent such as ethanol or tetrahydrofuran, or, preferably, in the absence of a solvent at a temperature of 20 to 100 °C; or

(b) treating an amide of general formula (IX):

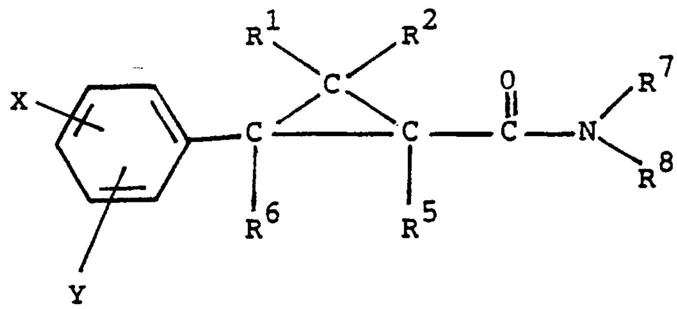

(IX)

wherein $R^1$, $R^2$, $R^5$, $R^6$, $R^7$, $R^8$, X and Y are as defined above with a reducing agent (usually lithium aluminium hydride) in a convenient solvent such as diethyl ether or tetrahydrofuran; if desired preparing the amides of general formula (IX) by reacting an acid chloride of general formula (X):

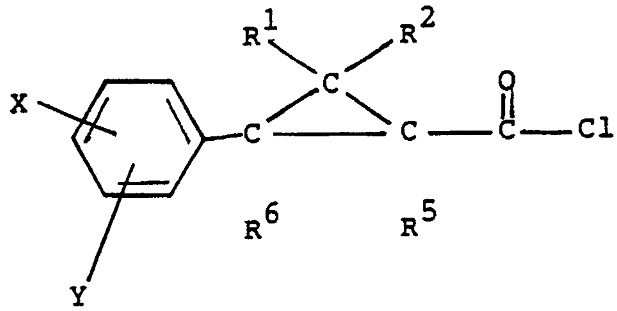

(X)

wherein $R^1$, $R^2$, $R^5$, $R^6$, X and Y are as defined with an amine of general formula (III):

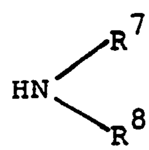

(III)

in a convenient solvent such as diethyl ether or tetrahydrofuran, the acid chloride of general formula (X) being prepared by reacting an acid of general formula (XI) :

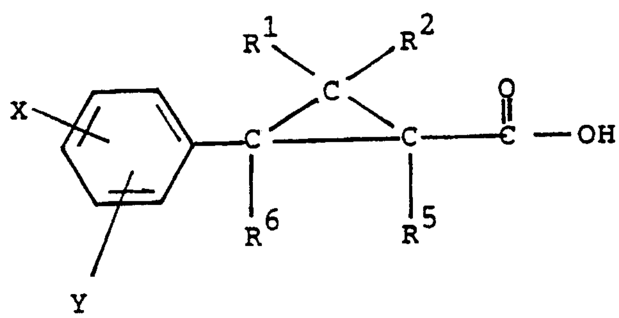

(XI )

wherein $R^1$, $R^2$, $R^5$, $R^6$, X and Y are as defined above with the usual chlorinating agents such as thionyl chloride or oxalyl chloride in a convenient solvent such as hexane or dichloromethane, the acid of general formula XI being itself prepared by hydrolysing the ester (V) in the normal manner using either sodium or potassium hydroxide in methanol/water solution at room temperature.

**2.** A process as claimed in claim 1 wherein the compounds prepared are of general formula (I):

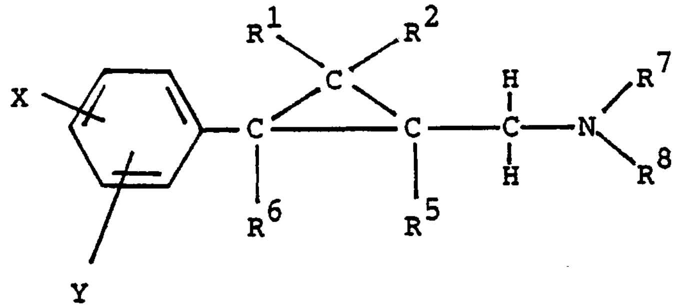 (I)

and stereoisomers and acid addition salts thereof, wherein $R^1$ and $R^2$ are hydrogen, methyl, or chlorine; $R^5$ and $R^6$ are hydrogen or methyl, $R^7$ and $R^8$ are piperidine, 3,5-dimethyl piperidine, morpholine, 2,6-dimethylmorpholine, diethylamino, 4-phenyl piperidine, 3-hydroxymethyl piperidine, thiomorpholine, piperazine, or 4-phenyl-piperazine; and X and Y are substituents at the 3- or 4-positions of the phenyl ring and one of which is hydrogen or $C_{1-4}$ alkyl, phenyl, phenoxy, benzyl, benzyloxy, or $C_{1-4}$ trialkylsilyl, provided that X and Y are not both hydrogen.

3. A process as claimed in claim 1 wherein the compounds have the formulae:

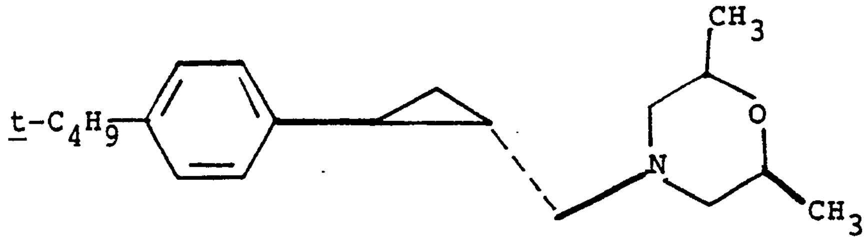

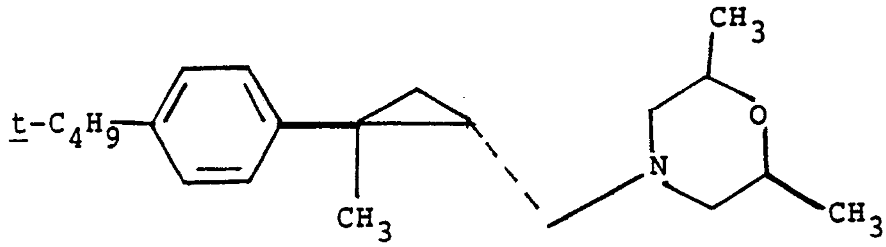

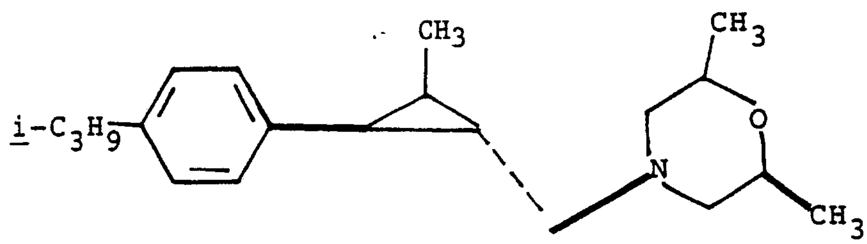

and

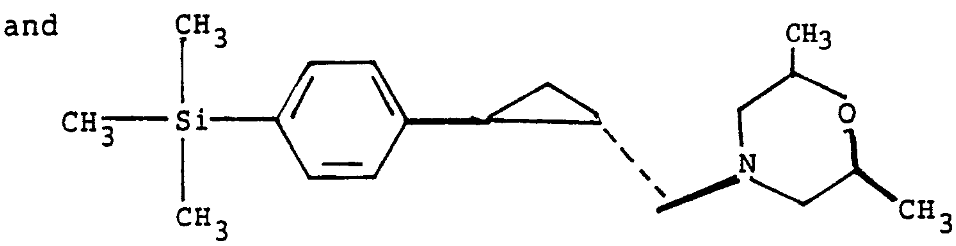

**Revendications**

1. Procédé pour combattre des champignons parasites de végétaux, qui consiste à appliquer à une plante, aux semences d'une plante ou bien au milieu dans lequel se trouve la plante ou les semences, un composé répondant à la formule générale (I) :

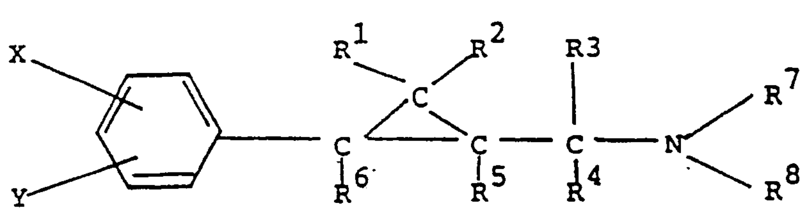

(I)

ou un de ses stéréo-isomères ou sels d'addition d'acides, formule dans laquelle $R^1$, $R^2$, $R^3$, $R^4$, $R^5$ et $R^6$, qui peuvent être identiques ou différents, représentent un atome d'hydrogène, un groupe alkyle

contenant 1 à 4 atomes de carbone ou un atome d'halogène ; $R^7$ et $R^8$, qui peuvent être identiques ou différents, représentent un atome d'hydrogène ou un groupe alkyle contenant 1 à 4 atomes de carbone ou forment conjointement un cycle qui peut contenir de l'oxygène, de l'azote ou du soufre comme hétéro-atome supplémentaire, cycle qui peut être facultativement substitué avec un ou plusieurs groupes alkyle en $C_1$ à $C_4$, ou bien un groupe phényle ou un groupe hydroxyalkyle ; X et Y, qui peuvent être identiques ou différents, représentent un atome d'hydrogène ou un atome d'halogène ou un groupe alkyle contenant 1 à 6 atomes de carbone, un groupe cycloalkyle contenant 3 à 6 atomes de carbone, un groupe alcényle ou alcynyle contenant chacun 2 à 6 atomes de carbone, un groupe phényle, un groupe benzyle, un groupe alkoxy contenant 1 à 6 atomes de carbone, un groupe phénoxy, un groupe benzyloxy ou un groupe de formule

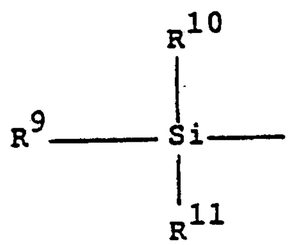

dans laquelle $R^9$, $R^{10}$ et $R^{11}$, qui peuvent être identiques ou différents, représentent un groupe alkyle contenant jusqu'à 4 atomes de carbone, un groupe alcényle ou alcynyle contenant chacun 2 à 4 atomes de carbone, un groupe cycloalkyle contenant 3 à 6 atomes de carbone, ou un groupe phényle.

2. Composés de formule générale (I) :

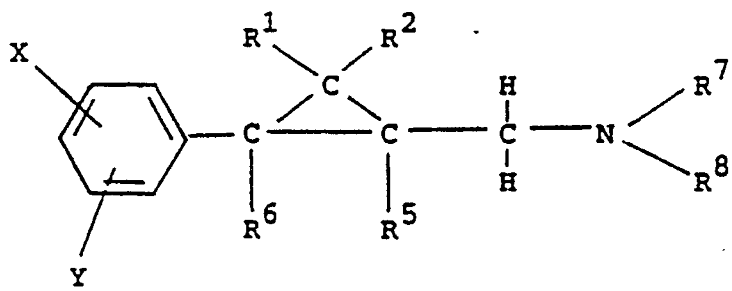

(I)

et leurs stéréo-isomères et sels d'addition d'acides, formule dans laquelle $R^1$ et $R^2$ représentent l'hydrogène, un halogène ou un groupe alkyle en $C_1$ à $C_4$ ; $R^5$ et $R^6$ représentent l'hydrogène ou un groupe alkyle en $C_1$ à $C_4$ ; $R^7$ et $R^8$, conjointement avec l'atome N adjacent, représentent un noyau pipéridine, morpholine, thiomorpholine ou pipérazine qui peut être substitué avec un groupe alkyle en $C_1$ à $C_4$, phényle ou hydroxyméthyle ; et X et Y représentent un groupe alkyle en $C_1$ à $C_4$, phényle, phénoxy, benzyle ou benzyloxy ou un groupe de formule

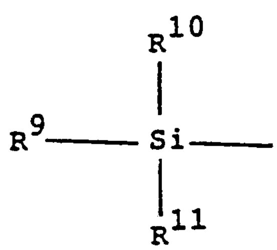

dans laquelle $R^9$, $R^{10}$ et $R^{11}$ représentent des groupes alkyle en $C_1$ à $C_4$.

3. Composés suivant la revendication 3 et répondant à la formule générale (I) :

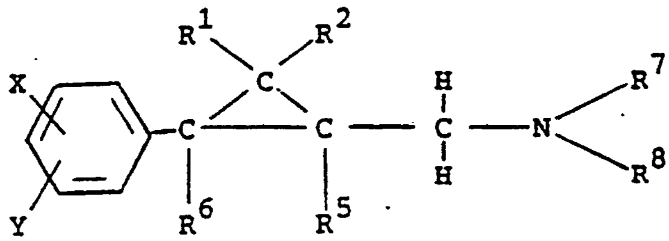

(I)

et leurs stéréo-isomères et sels d'addition d'acides, formule dans laquelle $R^1$ et $R^2$ représentent l'hydrogène, un groupe méthyle ou le chlore ; $R^5$ et $R^6$ représentent l'hydrogène ou un groupe méthyle ; $R^6$ et $R^7$ représentent un groupe pipéridine, 3,5-diméthylpipéridine, morpholine, 2,6-diméthylmorpholine, diéthylamino, 4-phénylpipéridine, 3-hydroxyméthylpipéridine, thiomorpholine, pipérazine ou 4-phénylpipérazine ; et X et Y sont des substituants en position 3 ou 4 du noyau phényle, dont l'un représente l'hydrogène ou un groupe alkyle en $C_1$ à $C_4$, tandis que l'autre représente l'hydrogène, un groupe alkyle en $C_1$ à $C_4$, phényle, phénoxy, benzyle, benzyloxy ou trialkylsilyle en $C_1$ à $C_4$, sous réserve que X et Y ne représentent pas l'un et l'autre l'hydrogène.

4. Composés de formules

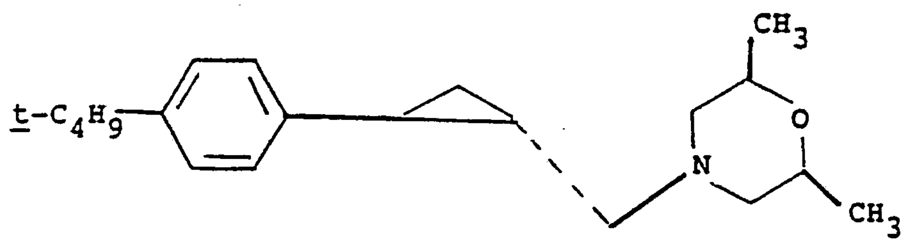

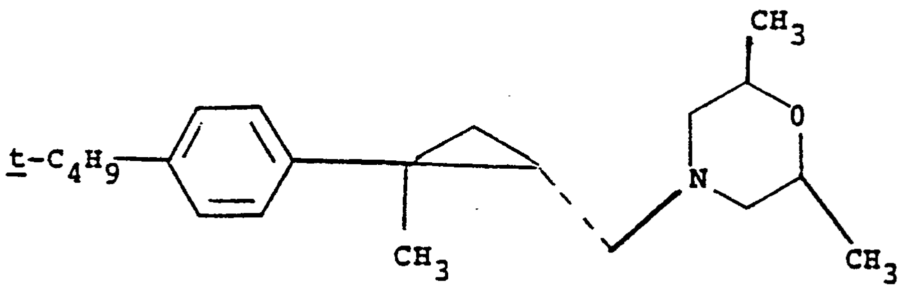

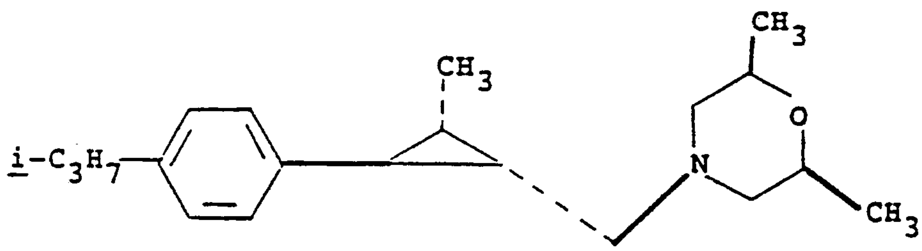

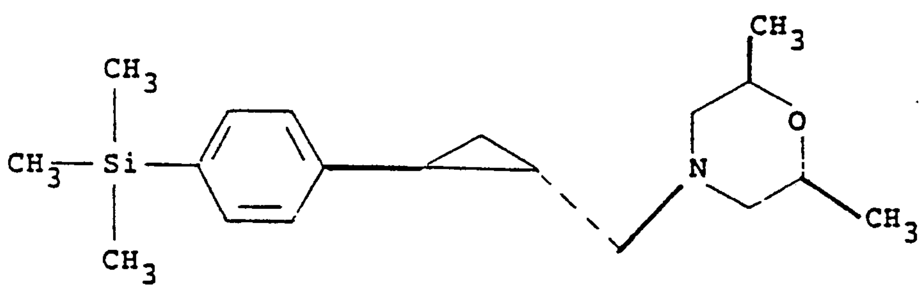

5. Procédé de préparation des composés répondant aux définitions suivant les revendications 2 à 4, qui consiste à traiter un composé de formule générale (II) :

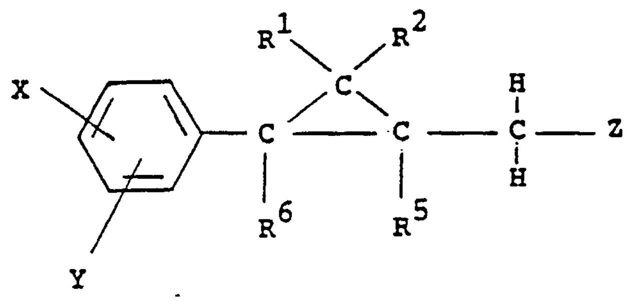

(II)

dans laquelle $R^1$, $R^2$, $R^5$, $R^6$, X et Y répondent aux définitions et Z représente un groupe partant tel que le chlore, le brome, un groupe mésylate ou tosylate, avec une amine de formule générale (III) :

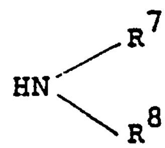

(III)

dans laquelle $R^7$ et $R^8$ répondent aux définitions précitées, en présence d'un solvant convenable tel que l'éthanol ou le tétrahydrofuranne ou bien, de préférence, en l'absence d'un solvant, à une température de 20 à 100° C.

6. Procédé de préparation des composés de formule générale (I) suivant les revendications 2 à 4, qui consiste à traiter un amide de formule générale (IX) :

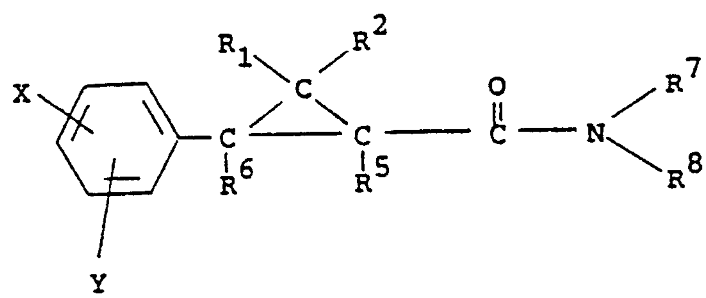

(IX)

dans laquelle $R^1$, $R^2$, $R^5$, $R^6$, $R^7$, $R^8$, X et Y répondent aux définitions, avec un agent réducteur (habituellement l'hydrure de lithium-aluminium) dans un solvant convenable, tel que l'éther diéthylique ou le tétrahydrofuranne.

7. Procédé suivant la revendication 6, qui consiste à préparer des amides de formule générale (IX) par réaction d'un chlorure d'acide de formule générale (X) :

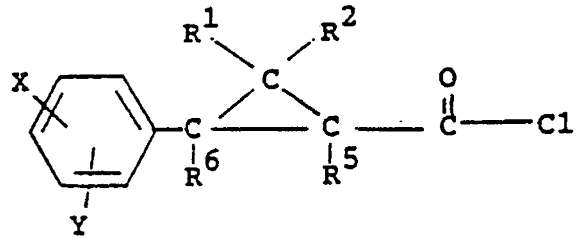

(X)

dans laquelle $R^1$, $R^2$, $R^5$, $R^6$, X et Y répondent aux définitions, avec une amine de formule générale (III) :

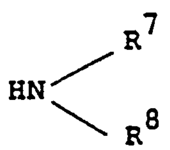

(III)

dans un solvant convenable, tel que l'éther diéthylique ou le tétrahydrofuranne, le chlorure d'acide de formule générale (X) étant préparé par réaction d'un acide de formule générale (XI) :

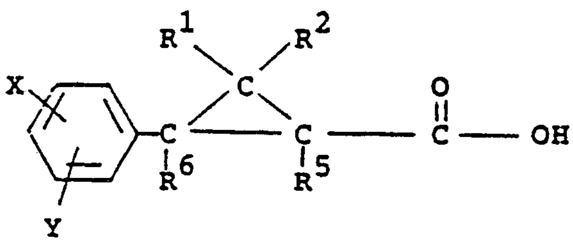

(XI)

dans laquelle $R^1$, $R^2$, $R^5$, $R^6$, X et Y répondent aux définitions précitées, avec les agents usuels de chloration tels que le chlorure de thionyle ou le chlorure d'oxalyle dans un solvant convenable tel que l'hexane ou le dichlorométhane, l'acide de formule générale XI étant lui-même préparé par hydrolyse de l'ester (V) de la manière habituelle au moyen d'hydroxyde de sodium ou de potassium dans une solution de méthanol/eau à température ambiante.

8. Composition fongicide comprenant un composé de formule générale (I) suivant l'une quelconque des revendications 2 à 4 ou un de ses sels d'addition d'acides ou stéréo-isomères ; et, facultativement, un support ou diluant.

9. Procédé pour combattre des champignons, qui consiste à appliquer à une plante, aux semences d'une plante ou au milieu dans lequel se trouve une plante ou ses semences, un composé, ou un de ses sels d'addition d'acides ou stéréo-isomères, suivant l'une quelconque des revendications 2 à 4 ou bien une composition suivant la revendication 8.

REVENDICATIONS pour l'Etat Contractant suivant: AT

1. Procédé de préparation de composés de formule générale (I) :

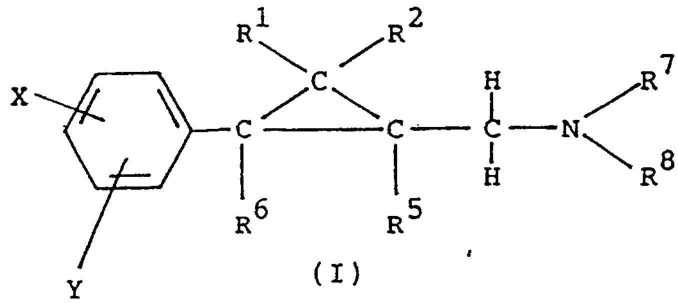

(I)

et de leurs stéréo-isomères et sels d'addition d'acides, formule dans laquelle $R^1$ et $R^2$ représentent l'hydrogène, un halogène ou un groupe alkyle en $C_1$ à $C_4$, $R^5$ et $R^6$ représentent l'hydrogène ou un groupe alkyle en $C_1$ à $C_4$ ; $R^7$ et $R^8$, conjointement avec l'atome N adjacent, représentent un noyau pipéridine, morpholine, thiomorpholine ou pipérazine qui peut être substitué avec un groupe alkyle en $C_1$ à $C_4$, phényle ou hydroxyméthyle ; et X et Y représentent un groupe alkyle en $C_1$ à $C_4$, phényle, phénoxy, benzyle, benzyloxy ou un groupe de formule

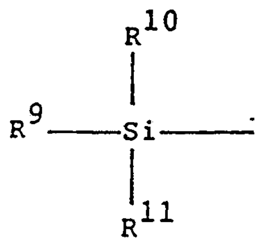

dans laquelle $R^9$, $R^{10}$ et $R^{11}$ représentent un groupe alkyle en $C_1$ à $C_4$, qui consiste :

(a) à traiter un composé de formule générale (II) :

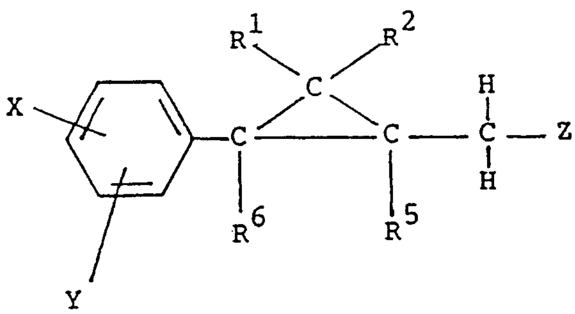

(II)

dans laquelle $R^1$, $R^2$, $R^5$, $R^6$, X et Y répondent aux définitions précitées et Z représente un groupe partant tel que le chlore, le brome, un groupe mésylate ou tosylate, avec une amine de formule (III) :

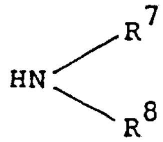

(III)

dans laquelle $R^7$ et $R^8$ répondent aux définitions précitées, en présence d'un solvant convenable tel que l'éthanol ou le tétrahydrofuranne ou, de préférence, en l'absence de solvant, à une température de 20 à 100 °C ; ou

(b) à traiter un amide de formule générale (IX) :

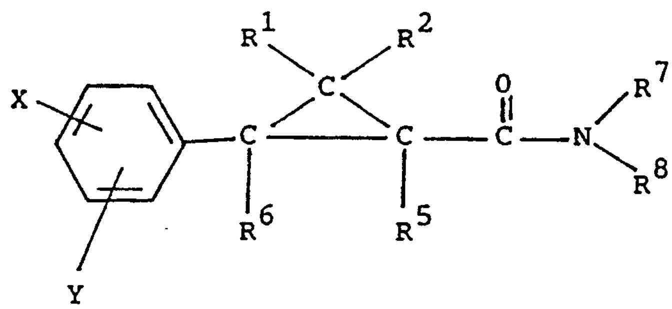

(IX)

dans laquelle $R^1$, $R^2$, $R^5$, $R^6$, $R^7$, $R^8$, X et Y répondent aux définitions précitées, avec un agent réducteur (habituellement l'hydrure de lithium-aluminium) dans un solvant convenable tel que l'éther diéthylique ou le tétrahydrofuranne ; le cas échéant en préparant les amides de formule générale (IX) par réaction d'un chlorure d'acide de formule générale (X) :

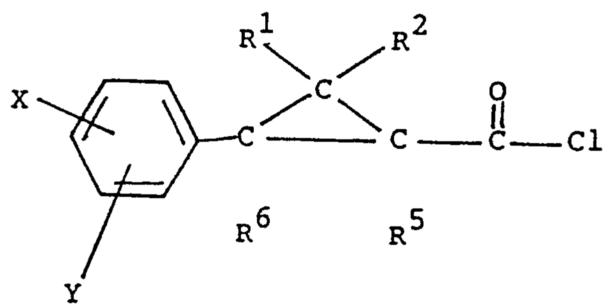

(X)

dans laquelle $R^1$, $R^2$, $R^5$, $R^6$, X et Y répondent aux définitions précitées, avec une amine de formule générale (III) :

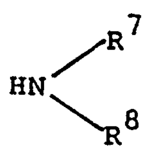

(III)

dans un solvant convenable tel que l'éther diéthylique ou le tétrahydrofuranne, le chlorure d'acide de formule générale (X) étant préparé par réaction d'un acide de formule générale (XI) :

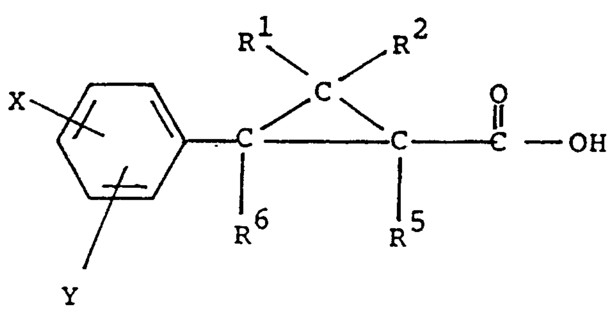

(XI )

dans laquelle $R^1$, $R^2$, $R^5$, $R^6$, X et Y répondent aux définitions précitées, avec les agents usuels de chloration tels que le chlorure de thionyle ou le chlorure d'oxalyle, dans un solvant convenable tel que l'hexane ou le dichlorométhane, l'acide de formule générale XI étant lui-même préparé par hydrolyse de l'ester (V) de la manière habituelle au moyen d'hydroxyde de sodium ou de potassium dans une solution de méthanol/eau à température ambiante.

2. Procédé suivant la revendication 1, dans lequel les composés préparés répondent à la formule générale (I) :

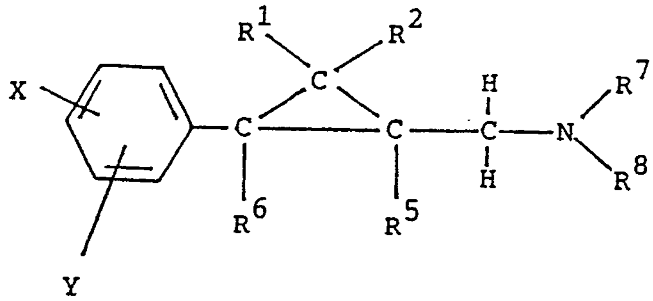

(I)

ainsi que leurs stéréo-isomères et sels d'addition d'acides, formule dans laquelle $R^1$ et $R^2$ représentent l'hydrogène, un groupe méthyle ou le chlore ; $R^5$ et $R^6$ représentent l'hydrogène ou un groupe méthyle, $R^7$ et $R^8$ représentent un groupe pipéridine, 3,5-diméthylpipéridine, morpholine, 2,6-diméthylmorpholine, diéthylamino, 4-phénylpipéridine, 3-hydroxyméthylpipéridine, thiomorpholine, pipérazine ou 4-phénylpipérazine ; et X et Y représentent des substituants en position 3 ou 4 du noyau phényle, dont l'un représente l'hydrogène ou un groupe alkyle en $C_1$ à $C_4$, phényle, phénoxy, benzyle, benzyloxy ou trialkylsilyle en $C_1$ à $C_4$, sous réserve que X et Y ne représentent pas l'un et l'autre l'hydrogène.

3. Procédé suivant la revendication 1, dans lequel les composés répondent aux formules :

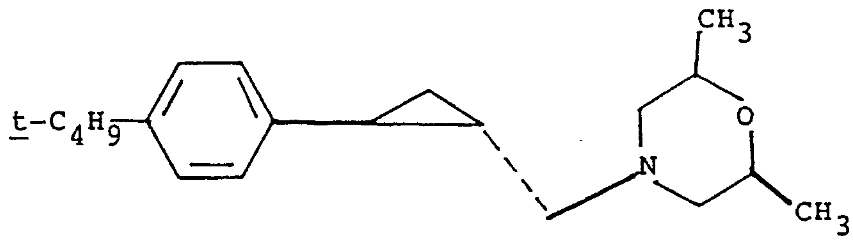

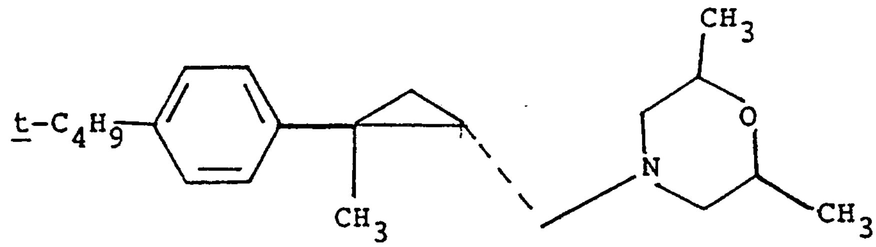

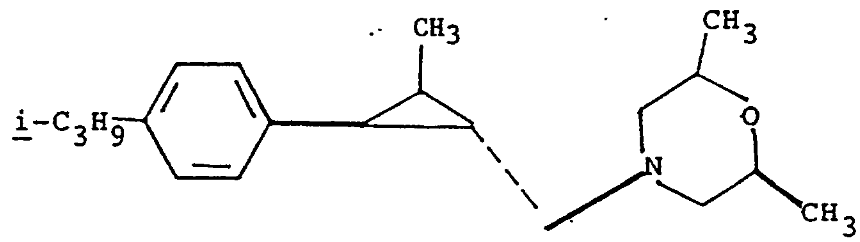

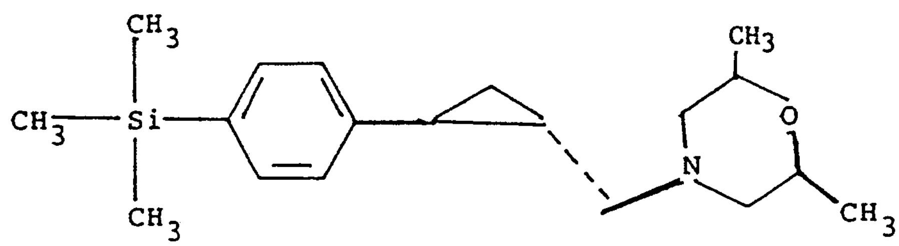

## Ansprüche

1. Verfahren zur Bekämpfung von Pflanzenpilzen, bei welchem auf eine Pflanze, auf den Samen einer Pflanze oder auf den Standort der Pflanze oder des Samens eine Verbindung der allgemeinen Formel (I)

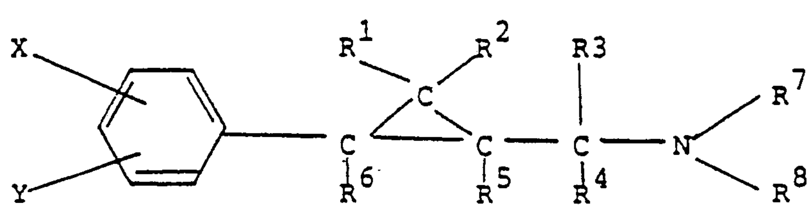

(I)

oder ein Stereoisomer oder Säureadditionssalze davon aufgebracht wird, worin $R^1$, $R^2$, $R^3$, $R^4$, $R^5$ und $R^6$, welche gleich oder verschieden sein können, für ein Wasserstoffatom oder eine Alkylgruppe mit 1

bis 4 Kohlenstoffatomen oder ein Halogenatom stehen; $R^7$ und $R^8$, welche gleich oder verschieden sein können, für ein Wasserstoffatom oder eine Alkylgruppe mit 1 bis 4 Kohlenstoffatomen stehen oder gemeinsam einen Ring bilden, der Sauerstoff, Stickstoff oder Schwefel als zusätzliches Heteroatom enthalten kann, wobei der Ring gegebenenfalls mit einer oder mehreren $C_{1-4}$-Alkylgruppen oder einer Phenylgruppe oder einer Hydroxyalkylgruppe substituiert sein kann; und X und Y, welche gleich oder verschieden sein können, für ein Wasserstoff- oder Halogenatom oder eine Alkylgruppe mit 1 bis 6 Kohlenstoffatomen, eine Cycloalkylgruppe mit 3 bis 6 Kohlenstoffatomen oder eine Alkenyl- oder Alkinylgruppe mit jeweils 2 bis 6 Kohlenstoffatomen, eine Phenylgruppe, eine Benzylgruppe, eine Alkoxygruppe mit 1 bis 6 Kohlenstoffatomen, eine Phenoxygruppe, eine Benzyloxygruppe oder eine Gruppe der Formel

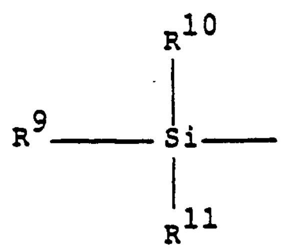

stehen, worin $R^9$, $R^{10}$ und $R^{11}$, welche gleich oder verschieden sein können, für eine Alkylgruppe mit bis zu 4 Kohlenstoffatomen, eine Alkenyl- oder Alkinylgruppe mit jeweils 2 bis 4 Kohlenstoffatomen, eine Cycloalkylgruppe mit 3 bis 6 Kohlenstoffatomen oder eine Phenylgruppe stehen.

2. Verbindungen der allgemeinen Formel (I)

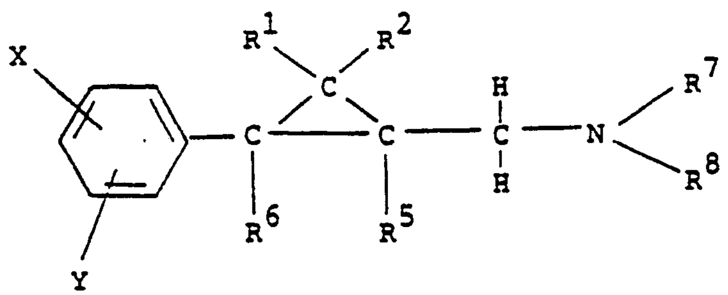

(I)

und Stereoisomere und Säureadditionssalze davon, worin $R^1$ und $R^2$ für Wasserstoff, Halogen oder $C_{1-4}$-Alkyl stehen; $R^5$ und $R^6$ für Wasserstoff oder $C_{1-4}$-Alkyl stehen; $R^7$ und $R^8$ gemeinsam mit dem N-Atom einen Piperidin-, Morpholin-, Thiomorpholin-oder Piperazin-Ring bilden, der mit $C_{1-4}$-Alkyl, Phenyl oder Hydroxymethyl substituiert sein kann; und X und Y für $C_{1-4}$-Alkyl, Phenyl, Phenoxy, Benzyl, Benzyloxy oder eine Gruppe der Formel

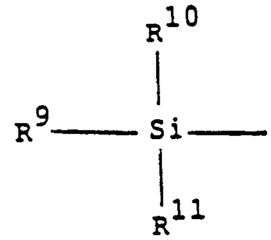

stehen, worin $R^9$, $R^{10}$ und $R^{11}$ für $C_{1-4}$-Alkyl stehen.

3. Verbindungen nach Anspruch 2 und der allgemeinen Formel (I)

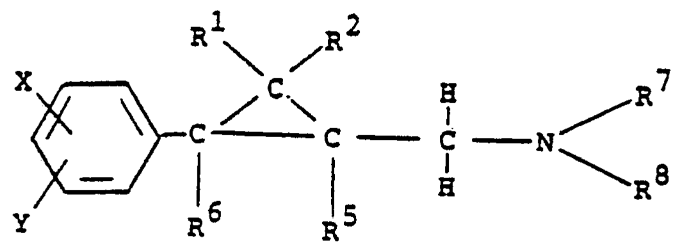

(I)

und Stereoisomere und Säureadditionssalze davon, worin $R^1$ und $R^2$ für Wasserstoff, Methyl oder Chlor stehen; $R^5$ und $R^6$ für Wasserstoff oder Methyl stehen; $R^7$ und $R^8$ für Piperidin, 3,5-Dimethylpiperidin, Morpholin, 2,6-Dimethylmorpholin, Diethylamino, 4-Phenylpiperidin, 3-Hydroxymethylpiperidin, Thiomorpholin, Piperazin oder 4-Phenylpiperazin stehen; und X und Y für Substituenten in der 3- oder 4-Stellung des Phenyl-Rings stehen, wobei einer davon Wasserstoff oder $C_{1-4}$-Alkyl ist, während der andere Wasserstoff, $C_{1-4}$-Alkyl, Phenyl, Phenoxy, Benzyl, Benzyloxy oder $C_{1-4}$-Trialkylsilyl ist, mit der Maßgabe, daß X und Y nicht beide Wasserstoff sind.

4. Die Verbindungen

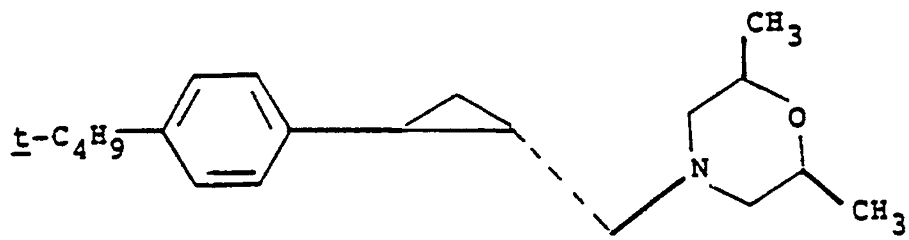

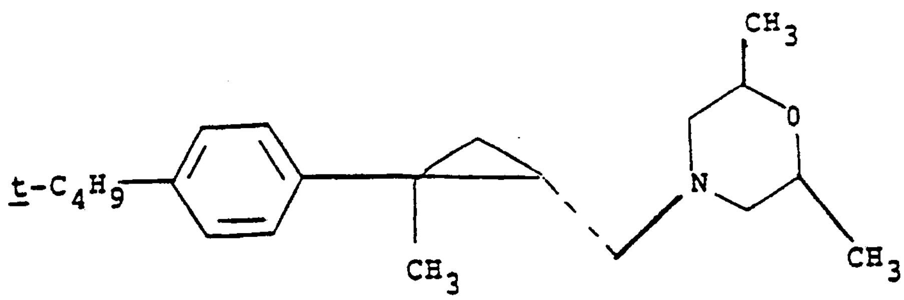

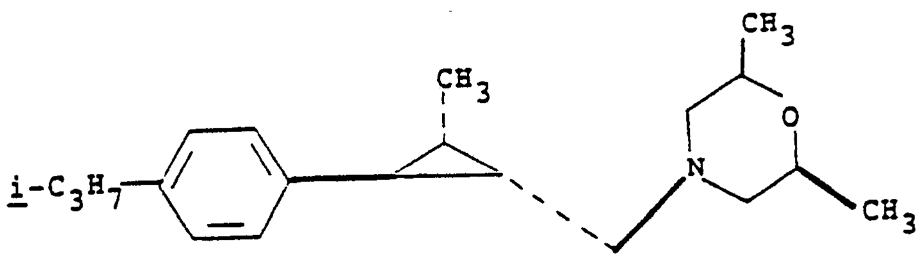

und

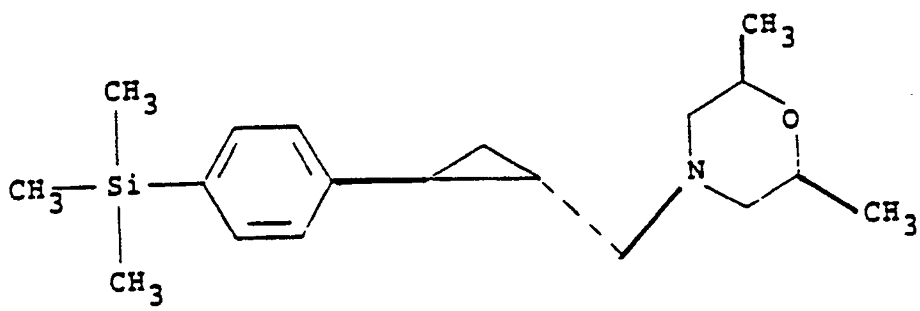

5. Verfahren zur Herstellung der Verbindungen, die in den Ansprüchen 2 bis 4 definiert sind, bei welchem eine Verbindung der allgemeinen Formel (II)

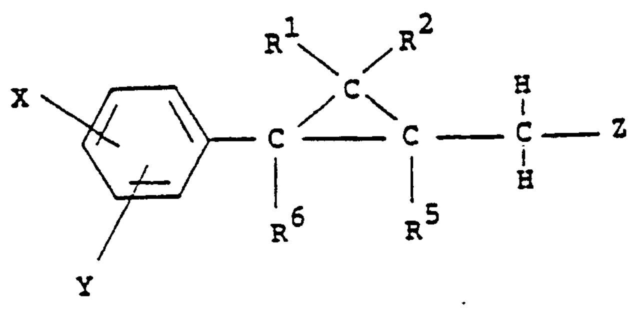

(II)

worin $R^1$, $R^2$, $R^5$, $R^6$, X und Y die angegebenen Bedeutungen besitzen und Z für eine abspaltbare Gruppe steht, wie z.B. Chlor, Brom, Mesylat oder Tosylat, mit einem Amin der allgemeinen Formel (III)

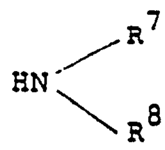

(III)

worin $R^7$ und $R^8$ die oben angegebenen Bedeutungen besitzen, in Gegenwart eines geeigneten Lösungsmittels, wie z.B. Ethanol oder Tetrahydrofuran, oder vorzugsweise in Abwesenheit eines Lösungsmittels bei einer Temperatur von 20 bis 100° C behandelt wird.

6. Verfahren zur Herstellung der Verbindungen der allgemeinen Formel (I), wie sie in den Ansprüchen 2 bis 4 definiert sind, bei welchem ein Amid der allgemeinen Formel (IX)

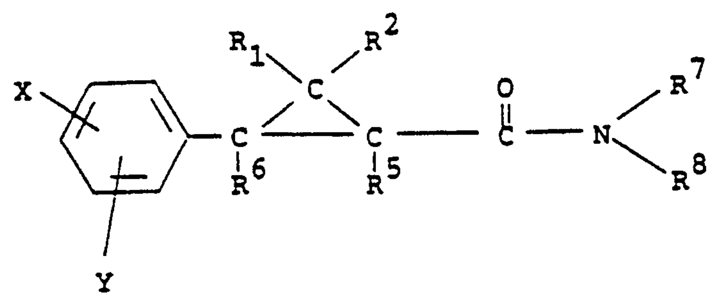

(IX)

worin $R^1$, $R^2$, $R^5$, $R^6$, $R^7$, $R^8$, X und Y die angegebenen Bedeutungen besitzen, mit einem Reduktionsmittel (üblicherweise Lithiumaluminiumhydrid) in einem geeigneten Lösungsmittel, wie z.B. Diethylether oder Tetrahydrofuran, behandelt wird.

7. Verfahren nach Anspruch 6, bei welchem Amide der allgemeinen Formel (IX) hergestellt werden, und zwar durch Umsetzung eines Säurechlorids der allgemeinen Formel (X)

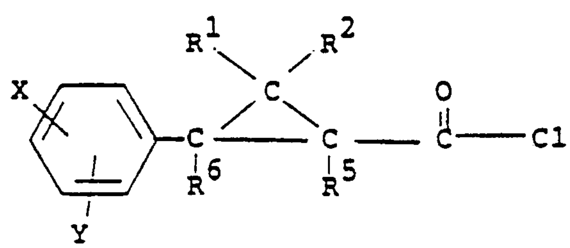

(X)

worin $R^1$, $R^2$, $R^5$, $R^6$, X und Y die angegebenen Bedeutungen besitzen, mit einem Amin der allgemeinen Formel (III)

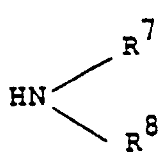

(III)

in einem geeigneten Lösungsmittel, wie z.B. Diethylether oder Tetrahydrofuran, wobei das Säurechlorid

der allgemeinen Formel (X) hergestellt wird durch Umsetzung einer Säure der allgemeinen Formel (XI)

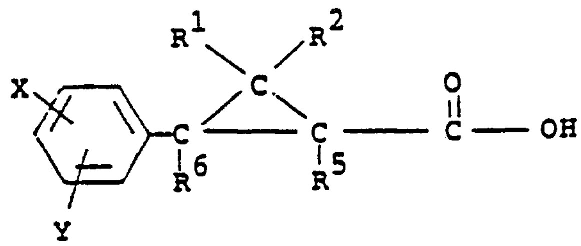 (XI)

worin $R^1$, $R^2$, $R^5$, $R^6$, X und Y die oben angegebenen Bedeutungen besitzen, mit einem üblichen Chlorierungsmittel, wie z.B. Thionylchlorid oder Oxalylchlorid, in einem geeigneten Lösungsmittel, wie z.B. Hexan oder Dichloromethan, wobei die Säure der allgemeinen Formel (XI) selbst hergestellt wird durch Hydrolyse des Esters (V) in üblicher Weise unter Verwendung von entweder Natrium- oder Kaliumhydroxid in einer Methanol/ Wasser-Lösung bei Raumtemperatur.

8. Fungicide Zusammensetzung, welche eine Verbindung der allgemeinen Formel (I), wie sie in einem der Ansprüche 2 bis 4 beansprucht wird, oder ein Säureadditionssalz oder ein Stereoisomer davon und gegebenenfalls ein Verdünnungs- oder Trägermittel enthält.

9. Verfahren zur Bekämpfung von Pilzen, bei welchem auf die Pflanze oder auf den Samen der Pflanze oder auf den Standort der Pflanze oder des Samens eine Verbindung oder ein Säureadditionssalz oder ein Stereoisomer davon nach einem der Ansprüche 2 bis 4 oder eine Zusammensetzung nach Anspruch 8 aufgebracht wird.

PATENTANSPRÜCHE für folgenden Vertragsstaat : AT

1. Verfahren zur Herstellung der Verbindungen der allgemeinen Formel (I)

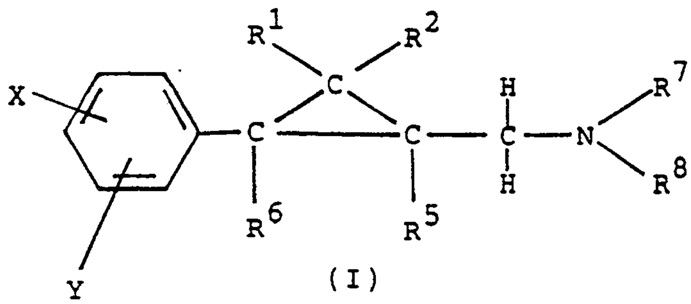

(I)

und der Stereoisomere und Säureadditionssalze davon, worin $R^1$ und $R^2$ für Wasserstoff, Halogen oder $C_{1-4}$-Alkyl stehen; $R^5$ und $R^6$ für Wasserstoff oder $C_{1-4}$-Alkyl stehen; $R^7$ und $R^8$ gemeinsam mit dem N-Atom einen Piperidin-, Morpholin-, Thiomorpholin-oder Piperazin-Ring bilden, der mit $C_{1-4}$-Alkyl, Phenyl oder Hydroxymethyl substituiert sein kann; und X und Y für $C_{1-4}$-Alkyl, Phenyl, Phenoxy, Benzyl, Benzyloxy oder eine Gruppe der Formel

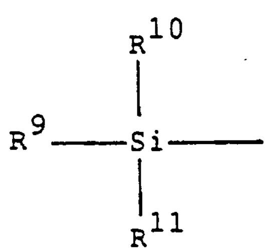

stehen, worin $R^9$, $R^{10}$ und $R^{11}$ für $C_{1-4}$-Alkyl stehen, bei welchem
(a) eine Verbindung der allgemeinen Formel (II)

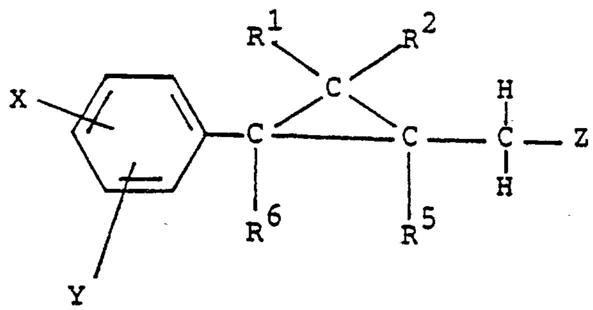

(II)

worin $R^1$, $R^2$, $R^5$, $R^6$, X und Y die oben angegebenen Bedeutungen besitzen und Z für eine abspaltbare Gruppe steht, wie z.B. Chlor, Brom, Mesylat oder Tosylat, mit einem Amin der allgemeinen Formel (III)

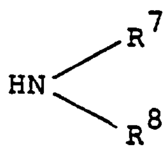

(III)

worin $R^7$ und $R^8$ die oben angegebenen Bedeutungen besitzen, in Gegenwart eines geeigneten Lösungsmittels, wie z.B. Ethanol oder Tetrahydrofuran, oder vorzugsweise in Abwesenheit eines Lösungsmittels bei einer Temperatur von 20 bis 100° C behandelt wird; oder

(b) ein Amid der allgemeinen Formel (IX)

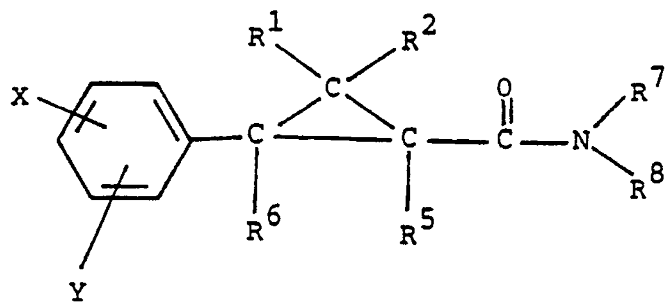

(IX)

worin $R^1$, $R^2$, $R^5$, $R^6$, $R^7$, $R^8$, X und Y die oben angegebenen Bedeutungen besitzen, mit einem Reduktionsmittel (üblicherweise Lithiumaluminiumhydrid) in einem geeigneten Lösungsmittel, wie z.B. Diethylether oder Tetrahydrofuran, behandelt wird, wobei gegebenenfalls die Amide der allgemeinen Formel (IX) hergestellt werden durch Umsetzung eines Säurechlorids der allgemeinen Formel (X)

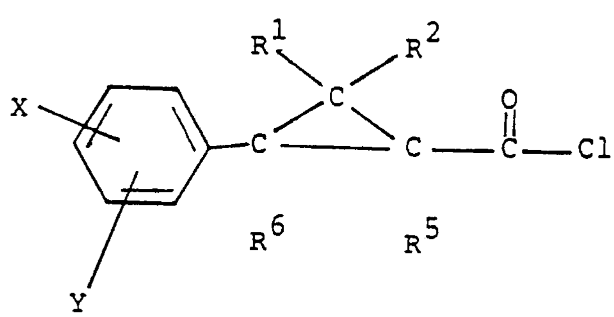

(X)

worin $R^1$, $R^2$, $R^5$, $R^6$, X und Y die angegebenen Bedeutungen besitzen, mit einem Amin der allgemeinen Formel (III)

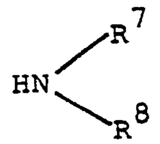

(III)

in einem geeigneten Lösungsmittel, wie z.B. Diethylether oder Tetrahydrofuran, wobei das Säurechlorid der allgemeinen Formel (X) hergestellt wird durch Umsetzung einer Säure der allgemeinen Formel (XI)

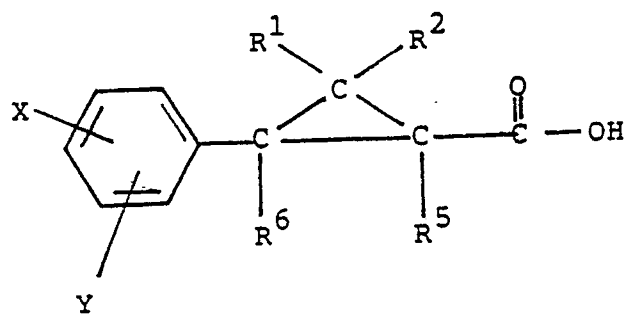

(XI)

worin $R^1$, $R^2$, $R^5$, $R^6$, X und Y die oben angegebenen Bedeutungen besitzen, mit einem üblichen Chlorierungsmittel, wie z.B. Thionylchlorid oder Oxalylchlorid, in einem geeigneten Lösungsmittel, wie z.B. Hexan oder Dichloromethan, wobei die Säure der allgemeinen Formel (XI) selbst hergestellt wird durch Hydrolyse des Esters (V) in üblicher Weise unter Verwendung von entweder Natrium- oder Kaliumhydroxid in einer Methanol/ Wasser-Lösung bei Raumtemperatur.

2. Verfahren nach Anspruch 1, bei welchem die hergestellten Verbindungen Verbindungen der allgemeinen Formel (I)

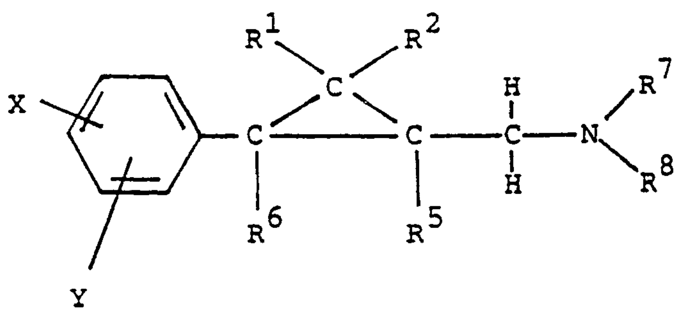

(I)

und Stereoisomere und Säureadditionssalze davon sind, worin $R^1$ und $R^2$ für Wasserstoff, Methyl oder Chlor stehen; $R^5$ und $R^6$ für Wasserstoff oder Methyl stehen; $R^7$ und $R^8$ für Piperidin, 3,5-Dimethylpiperidin, Morpholin, 2,6-Dimethylmorpholin, Diethylamino, 4-Phenylpiperidin, 3-Hydroxymethylpiperidin, Thiomorpholin, Piperazin oder 4-Phenylpiperazin stehen; und X und Y für Substituenten in der 3- oder 4-Stellung des Phenyl-Rings stehen, wobei einer davon Wasserstoff oder $C_{1-4}$-Alkyl ist, während der andere Wasserstoff, $C_{1-4}$-Alkyl, Phenyl, Phenoxy, Benzyl, Benzyloxy oder $C_{1-4}$-Trialkylsilyl ist, mit der Maßgabe, daß X und Y nicht beide Wasserstoff sind.

3. Verfahren nach Anspruch 1, bei welchem die Verbindungen eine der folgenden Formeln aufweisen:

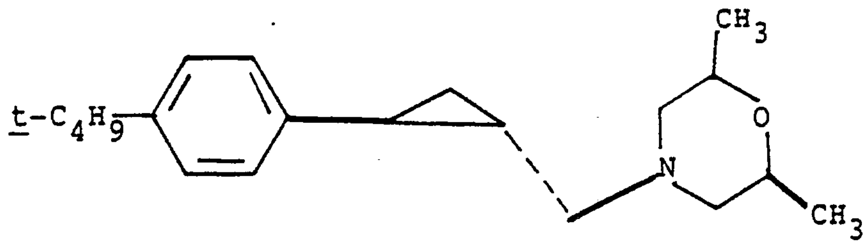

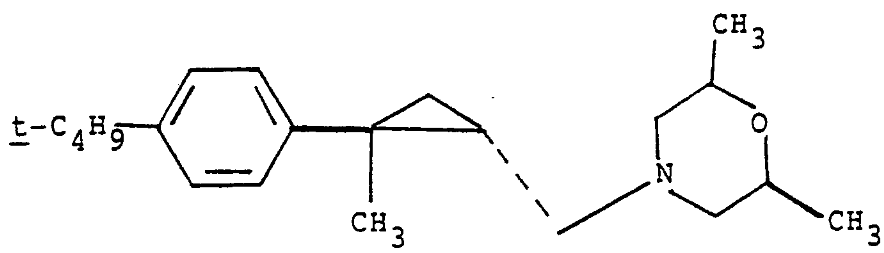

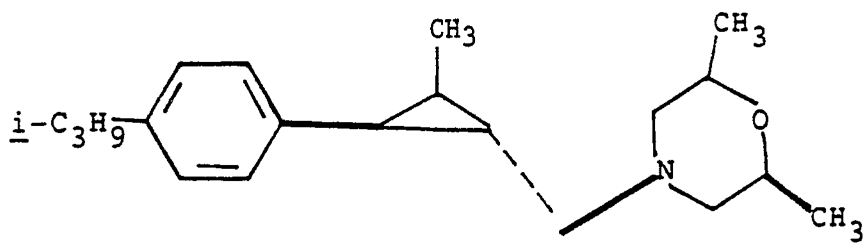

und